# EUROPEAN PATENT APPLICATION

(11) **EP 4 148 143 A1**
(43) Date of publication of application: **15.03.2023**
(21) Application number: 21382818.9
(22) Date of filing: 13.09.2021
(51) Int. Cl.: C12Q 1/6816, C12Q 1/70

(54) **POLYPURINE REVERSE HOOGSTEEN HAIRPINS AND PARALLEL CLAMPS AND THEIR USE AS BIOSENSORS**

(71) Applicant: Consejo Superior De Investigaciones Científicas (CSIC), 28006 Madrid (ES); Universitat de Barcelona, 08028 Barcelona (ES); Consorcio Centro de Investigación Biomédica en Red M.P., 28029 Madrid (ES)
(72) Inventor: MARTÍNEZ DE LA FUENTE, Jesús, 50018 Zaragoza (ES); CUESTAS AYLLÓN, Carlos, 50018 Zaragoza (ES); GRAZÚ BONAVÍA, María Valeria, 50018 Zaragoza (ES); FERNÁNDEZ SÁNCHEZ, César, 08193 Barcelona (ES); GUTIÉRREZ CAPITÁN, Manuel, 08193 Barcelona (ES); BALDI COLL, Antonio, 08193 Barcelona (ES); ERITJA CASADELLÀ, Ramon, 08034 Barcelona (ES); MARCO COLÁS, María Pilar, 08034 Barcelona (ES); VILAPLANA HOLGADO, María Luisa, 08034 Barcelona (ES); AVIÑÓ ANDRÉS, Ana María, 08034 Barcelona (ES); CIUDAD GÓMEZ, Carlos Julián, 08028 Barcelona (ES); NOÉ MATA, Verónica, 08028 Barcelona (ES)
(74) Representative: Hoffmann Eitle

(57) **Abstract**

The present invention relates to an *in vitro* method for detecting at least one target polynucleotide in a test sample, wherein the method comprises the steps of contacting the test sample with a first oligonucleotide and a second oligonucleotide and detecting the hybridization between the first and the second oligonucleotides to the at least one target polynucleotide. The present invention also relates to devices or systems to implement the method, and kits of parts comprising said oligonucleotides.

## Description

### TECHNICAL FIELD

The present invention relates to the field of biotechnology. Particularly, the present invention relates to the field of biosensors, more specifically to the field of biosensors for detecting target polynucleotides in a test sample.

### BACKGROUND ART

Several approaches have been developed to target polynucleotides of interest, such as antisense oligonucleotides, small interfering RNAs, and triplex-forming oligonucleotides (TFOs).

In TFOs, triple helices can be formed when oligonucleotides bind in a sequence-specific manner to the major groove of the double-stranded DNA target. TFOs are classified, at least, into three main categories that differ in their sequence composition and its orientation with respect to the polypurine Watson-Crick target strand. In the first class, called parallel triplexes, or the pyrimidine motif, the polypyrimidine third strand (C,T-oligonucleotide) binds in a parallel orientation to the polypurine strand of the duplex by Hoogsteen hydrogen bonds, forming T•A#T and C•G#C+ triads. In the second class, named antiparallel triplexes or Polypurine reverse Hoogsteen hairpins (PPRHs), the purine motif, the polypurine third strand (G,A-oligonucleotide) binds in an antiparallel orientation to the polypurine strand of the duplex by reverse Hoogsteen hydrogen bonds, forming T•A#A and C•G#G triads. Lastly, a third class, the third strand (G,T-oligonucleotide) binds either in a parallel or antiparallel orientation to the polypurine strand of the duplex, depending on the base sequence, forming T•A#T and C•G#G triads.

Double-stranded TFOs can be obtained by linking one of the Watson-Crick strands to one of the triplex forming polypurine or polypyrimidine strands. When linking the polypurine Watson-Crick strand to the Hoogsteen C,T-oligonucleotide strand a duplex parallel clamp is obtained. When the polypurine Watson-Crick strand is linked to the Hoogsteen G,A-oligonucleotide strand a PPRHs is obtained. Duplex parallel clamps and PPRHs, due to the intramolecular linkage of Hoogsteen bonds or reverse-Hoogsteen bonds between the two strands, form stable hairpin structures that are able to bind in a sequence-specific manner to a polypyrimidine rich-region in the target DNA

(that can be either double-stranded (ds) or single-stranded (ss)) producing a triplex structure and displacing the polypurine strand in the case of dsDNA. PPRH are oligonucleotides containing two polypurine domains, in a mirror repeat fashion, linked by single-stranded connector, forming double-stranded DNA stem-loop molecules. The two polypurine domains interact by intramolecular reverse-Hoogsteen bonds allowing the formation of this specific hairpin structure. PPRHs can be designed for virtually any region in the genome by searching for polypyrimidine stretches in the sequence of the desired target region.

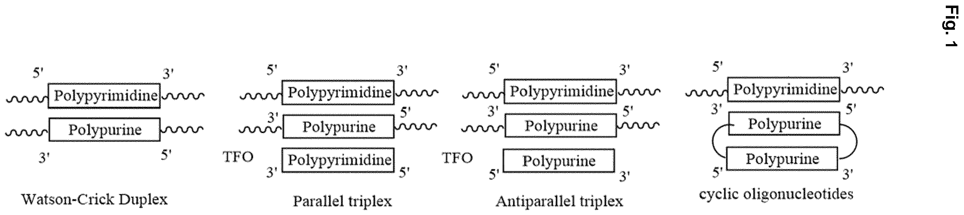

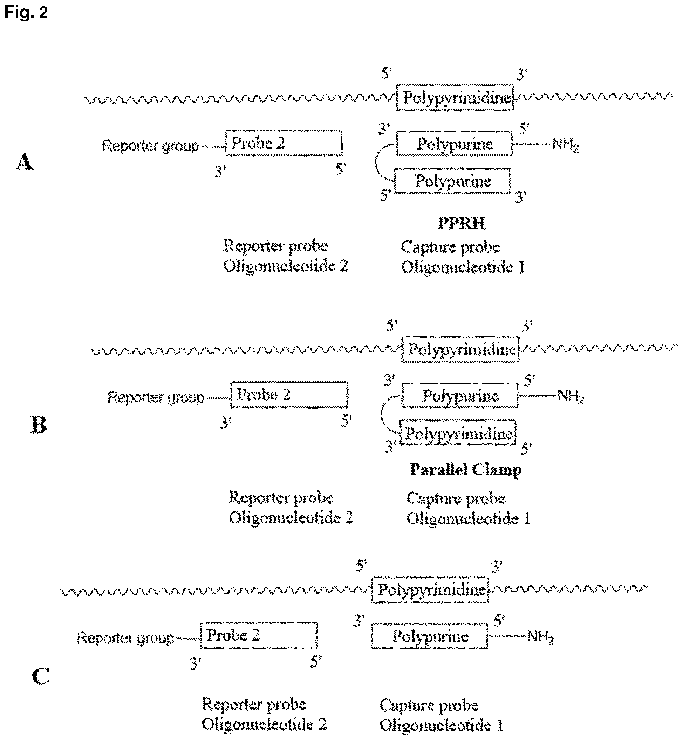

Although, in general, single stranded-TFOs and circular oligonucleotides (Fig. 1) have been widely used in different therapeutically approaches, they have been found to have some stability and specificity problems or, in the case of circular oligonucleotides, are difficult to prepare. Further, methods using PPRH and parallel clamps oligonucleotides as biosensors are still immature since, although these nucleotides are able to efficiently bind to target nucleotides, the translation of this binding into an efficient detecting system that provides the required sensitivity has been a limiting step to further develop these molecules to detect target polynucleotides. In the present invention, PPRH and parallel clamps (Fig. 2 A and B) are used in a several *in vitro* method for detecting target nucleotides in a very sensitive and efficient way.

### SUMMARY OF INVENTION

In a **first aspect,** the present invention relates to an *in vitro* method for detecting at least one target polynucleotide in a test sample, preferably in an isolated biological sample, comprising the steps of:
i) contacting the test sample with a first oligonucleotide, wherein the first oligonucleotide comprises a first polypurine region and a second region, wherein said first and second regions are connected by a linker molecule, and wherein:
   a) the second region is a polypurine region and said first and second regions are capable of forming a hairpin maintained by reverse-Hoogsteen bonds with the two strands running in antiparallel orientation, or
   b) the second region is a polypyrimidine region and said first and second regions are capable of forming a hairpin maintained by Hoogsteen bonds with the two strands running in parallel orientation;
   wherein the first region is of at least 12 nucleotides in length and wherein at least 6 nucleotides of the at least 12 nucleotides of the first region are engaged forming said hairpin with the second region; and
   wherein the first region is substantially complementary to a polypyrimidine target region comprised in the at least one target polynucleotide, wherein the hybridization between the first polypurine region and the polypyrimidine target region forms a structure comprising a triplex; and
ii) contacting the test sample with a second oligonucleotide, wherein the second oligonucleotide is substantially complementary to the at least one target polynucleotide; and
iii) detecting the hybridization between the first and the second oligonucleotides to the at least one target polynucleotide.

In an embodiment according to the first aspect, the second region is a polypurine region and the first and second polypurine regions are capable of forming a hairpin maintained by reverse-Hoogsteen bonds with the two strands running in antiparallel orientation, wherein the linker molecule that connects the first and the second polypurine regions is a single-stranded nucleotide region.

In an embodiment according to the first aspect or any of its embodiments, the first and the second polypurine regions comprised in the first oligonucleotide are identical in length.

In an embodiment according to the first aspect or any of its embodiments, the single-stranded nucleotide region comprised in the first oligonucleotide connects the 3'-end of the first polypurine region and the 5'-end of the second polypurine region or the single-stranded nucleotide region connects the 5'-end of the first polypurine region and the 3'-end of the polypurine second region.

In an embodiment according to the first aspect or any of its embodiments, the single-stranded nucleotide region comprised in the first oligonucleotide is a thymidine-rich region and said thymidine-rich region preferably consists of between 3 to 5 thymidines.

In an embodiment according to the first aspect or any of its embodiments, the first polypurine region comprised in the first oligonucleotide is 100% complementary to the polypyrimidine target region comprised in the at least one target polynucleotide.

In an embodiment according to the first aspect or any of its embodiments, the total length of the first oligonucleotide is between 21 to 100 nucleotides.

In an embodiment according to the first aspect or any of its embodiments, the total length of the second oligonucleotide is of at least 12 nucleotides.

In an embodiment according to the first aspect or any of its embodiments, the detection step iii) is performed by means of a reporter molecule, wherein the reporter molecule is conjugated to the first or to the second oligonucleotide.

In an embodiment according to the first aspect or any of its embodiments, the reporter molecule allows the detection to be carried out by means of, including but not limited to, radioactive, colorimetric, fluorescence, thermal dissipation, or electrochemical methods.

In an embodiment according to the first aspect or any of its embodiments, the second oligonucleotide is conjugated to a reporter molecule, wherein said reporter molecule consists of nanoparticles that are capable of emitting heat by surface plasmon resonance upon stimulation by a light source thereby allowing the detection of step iii) to be carried out by means of thermal dissipation detection, and wherein the first oligonucleotide is conjugated to a capture tag.

In an embodiment according to the first aspect or any of its embodiments, the first oligonucleotide is conjugated to a reporter molecule, wherein said reporter molecule consists of nanoparticles that are capable of emitting heat by surface plasmon resonance upon stimulation by a light source thereby allowing the detection of step iii) to be carried out by means of thermal dissipation detection, and wherein the second oligonucleotide is conjugated to a capture tag.

In an embodiment according to the first aspect or any of its embodiments, the method is performed using a thermo lateral flow system or device (thermo LFA) and the capture tag is used to bind the first or the second oligonucleotide to the pad of the lateral flow.

In an embodiment according to the first aspect or any of its embodiments, the second oligonucleotide is conjugated to a reporter molecule, wherein the reporter molecule is an enzyme that produces an electroactive product when an appropriate enzyme substrate is applied thereby allowing the detection of step iii) to be carried out by electrochemical detection.

In an embodiment according to the first aspect or any of its embodiments, the method is performed using an electrochemical system or device.

In an embodiment according to the first aspect or any of its embodiments, the first oligonucleotide is conjugated to a capture tag that is used to bind said first oligonucleotide to the surface of magnetic nanoparticles.

In an embodiment according to the first aspect or any of its embodiments, the second oligonucleotide is conjugated to a reporter molecule, wherein the reporter molecule comprises one or more fluorophores thereby allowing the detection of step iii) to be carried out by exciting the fluorophores with a laser beam of a defined wavelength.

In an embodiment according to the first aspect or any of its embodiments, the method is performed using a DNA microarray chip system or device.

In an embodiment according to the first aspect or any of its embodiments, the first oligonucleotide is conjugated to a capture tag that is used to bind said first oligonucleotide to the surface of a DNA microarray chip system or device.

In an embodiment according to the first aspect or any of its embodiments, the at least one target polynucleotide detected is a viral polynucleotide.

In an embodiment according to the first aspect or any of its embodiments, the viral polynucleotide is single-stranded, preferably RNA single-stranded.

In an embodiment according to the first aspect or any of its embodiments, the viral polynucleotide is selected from the group consisting of coronavirus, Influenza virus, or Respiratory syncytial virus genomes, or a combination thereof.

In an embodiment according to the first aspect or any of its embodiments, the at least one target polynucleotide is from SARS-CoV-2 virus, and the first and the second oligonucleotides comprise, respectively, a sequence selected from the list consisting of:
a) SEQ ID NOs: 60 (first oligonucleotide) and 5 (second oligonucleotide), or a sequence with at least 90% sequence identity over the full length with SEQ ID NOs: 60 and 5,
b) SEQ ID NOs: 63 (first oligonucleotide) and 13 (second oligonucleotide) or 12 (second oligonucleotide), or a sequence with at least 90% sequence identity over the full length with SEQ ID NOs: 63 and 13 or 12, or
c) SEQ ID NOs: 64 (first oligonucleotide) and 19 (second oligonucleotide), or a sequence with at least 90% sequence identity over the full length with SEQ ID NOs: 64 and 19.

In an embodiment according to the first aspect or any of its embodiments, the at least one target polynucleotide is from Influenza H1N1 virus, and the first and the second oligonucleotides comprise, respectively, a sequence selected from the list consisting of:
a) SEQ ID NOs: 67 (first oligonucleotide) and 33 (second oligonucleotide), or a sequence with at least 90% sequence identity over the full length with SEQ ID NOs: 67 and 33, or
b) SEQ ID NOs: 68 (first oligonucleotide) and 34 (second oligonucleotide), or a sequence with at least 90% sequence identity over the full length with SEQ ID NOs: 68 and 34.

In an embodiment according to the first aspect or any of its embodiments, the at least one target polynucleotide is from RSV strain B, and the first and the second oligonucleotides comprise, respectively, a sequence selected from the list consisting of:
a) SEQ ID NOs: 65 (first oligonucleotide) and 25 (second oligonucleotide), or a sequence with at least 90% sequence identity over the full length with SEQ ID NOs: 65 and 25, or
b) SEQ ID NOs: 66 (first oligonucleotide) and 26 (second oligonucleotide), or a sequence with at least 90% sequence identity over the full length with SEQ ID NOs: 66 and 26.

In an embodiment according to the first aspect or any of its embodiments, the test sample is an isolated biological sample, more preferably selected from the group consisting of saliva, mucus, nasopharyngeal swabs or washings from bodily cavities.

In an embodiment according to the first aspect or any of its embodiments, the *in vitro* method further comprises a step iv) of quantifying the amount of target polynucleotide present in the test sample.

In a second aspect, the present invention relates to a system or device for detecting a target polynucleotide in a test sample, preferably an isolated biological sample, comprising:
i) a first oligonucleotide, wherein the first oligonucleotide comprises a first polypurine region and a second region, wherein said first and second regions are connected by a single-stranded nucleotide region, and wherein:
   a) the second region is a polypurine region and said first and second regions are capable of forming a hairpin maintained by reverse-Hoogsteen bonds with the two strands running in antiparallel orientation, or
   b) the second region is a polypyrimidine region and said first and second regions are capable of forming a hairpin maintained by Hoogsteen bonds with the two strands running in parallel orientation;

   wherein the first region is of at least 12 nucleotides in length and wherein at least 6 nucleotides of the at least 12 nucleotides of the first region are engaged forming said hairpin with the second region; and
   wherein the first region is substantially complementary to a polypyrimidine target region comprised in the at least one target polynucleotide, wherein the hybridization between the first polypurine region and the polypyrimidine target region forms a structure comprising a triplex; and
ii) a second oligonucleotide, wherein the second oligonucleotide is substantially complementary to the at least one target polynucleotide; and
iii) optionally, a reporter molecule.

In a **third aspect,** the present invention relates to a kit of parts for detecting a target polynucleotide in a test sample, preferably an isolated biological sample, comprising at least two recipients comprising:
i) a first oligonucleotide, wherein the first oligonucleotide comprises a first polypurine region and a second region, wherein said first and second regions are connected by a single-stranded nucleotide region, and wherein:
   a) the second region is a polypurine region and said first and second regions are capable of forming a hairpin maintained by reverse-Hoogsteen bonds with the two strands running in antiparallel orientation, or
   b) the second region is a polypyrimidine region and said first and second regions are capable of forming a hairpin maintained by Hoogsteen bonds with the two strands running in parallel orientation;

   wherein the first region is of at least 12 nucleotides in length and wherein at least 6 nucleotides of the at least 12 nucleotides of the first region are engaged forming said hairpin with the second region; and
   wherein the first region is substantially complementary to a polypyrimidine target region comprised in the at least one target polynucleotide, wherein the hybridization between the first polypurine region and the polypyrimidine target region forms a structure comprising a triplex; and
ii) a second oligonucleotide, wherein the second oligonucleotide is substantially complementary to the at least one target polynucleotide; and
iii) optionally, a reporter molecule.

In a **fourth aspect,** the present invention relates to an *in vitro* use of the system or the kit as defined in the second or the third aspect or any of their embodiments, for detecting a target polynucleotide in a test sample, preferably in an isolated biological sample.

In an embodiment according to the fourth aspect or any of its embodiments the use is for implementing the method as defined in the first aspect or any of its embodiments.

In an embodiment according to the fourth aspect or any of its embodiments, the at least one target polynucleotide is a viral polynucleotide and is single stranded, preferably RNA single-stranded.

In an embodiment according to the fourth aspect or any of its embodiments, the viral polynucleotide is selected from the group consisting of coronavirus, Influenza virus or RSV genomes.

### BRIEF DESCRIPTION OF DRAWINGS

**Fig. 1****:** Schematic representation of an antiparallel natural duplex, a parallel triplex, an antiparallel triplex formed by a single triplex-forming oligonucleotide (TFO) and an antiparallel triplex formed by a cyclic oligonucleotide indicating the polarity (3'-5' or 5'-3') of the strands
**Fig. 2****:** Schematic representation of the binding location of the two oligonucleotides described in this document. **A)** Distribution of reporter probe and PPRH as capture probe (for example CC1-PPRH-amino), **B)** Distribution of reporter probe and a parallel clamp as capture probe (for example AA1-Parallelclamp-amino), **C)** Distribution of reporter probe and duplex capture probe (for example CC1-duplex-amino).
**Fig. 3****:** Design of the oligonucleotides involved in the CC1 system using CC1-PPRH-amino as capture probe.
**Fig. 4****:** Design of the oligonucleotides involved in the CC1 system using AA1-parallel clamp-amino as capture probe.
**Fig. 5****:** Bindings of PPRHs CC-1 and CC-3 to DNA and RNA probes corresponding to targets within Replicase and Spike, respectively, of the SARS-CoV-2 virus.
**Fig. 6****:** Bindings of PPRHs Hp-PB1 and Hp-NEP to DNA probes corresponding to targets within Segment 2 and 8, respectively, of the Influenza virus.
**Fig. 7****:** Bindings of PPRHs Hp-HRSV-1 and Hp-HRSV-V to DNA probes corresponding to targets within the L-protein upon infection and in the virion (protein N), respectively, of the Human Respiratory Syncytial virus.
**Fig. 8****:** Schematic representation of **A)** An analyte recognition/detection assay mediated by a Gold Nanoprism functionalized with capture probes and a NIR laser by using the system 1 **A.1)** and/or the system 2 **A.2), B)** Thermo-lateral flow assay diagram with the different components involved.
**Fig. 9****:** Thermo-Lateral Flow Assay results **A)** Front side of the cassettes and **B)** Back side of the cassettes with a positive and a negative sample using the system 1 to detect viral's genetic material; **C)** Front side of the cassettes and **D)** Back side of the cassettes with a positive and a negative sample using the system 2 and **E)** using the system 1 and samples with different viral load.
**Fig. 10****:** Comparison between **A)** PPRH and **B)** parallel clamps in the detection of SARS-COV-2 synthetic DNA samples at pH 6 and 7.2 The capture probes used in this example was CC1-PPRH-amino and AA1-parallel clamp-amino, the reporter probe was CC1biotine-RP and CC1 target.
**Fig. 11****:** Scheme of the microarray system or device to detect SARS-CoV-2 viral RNA by using PPRH oligonucleotides.

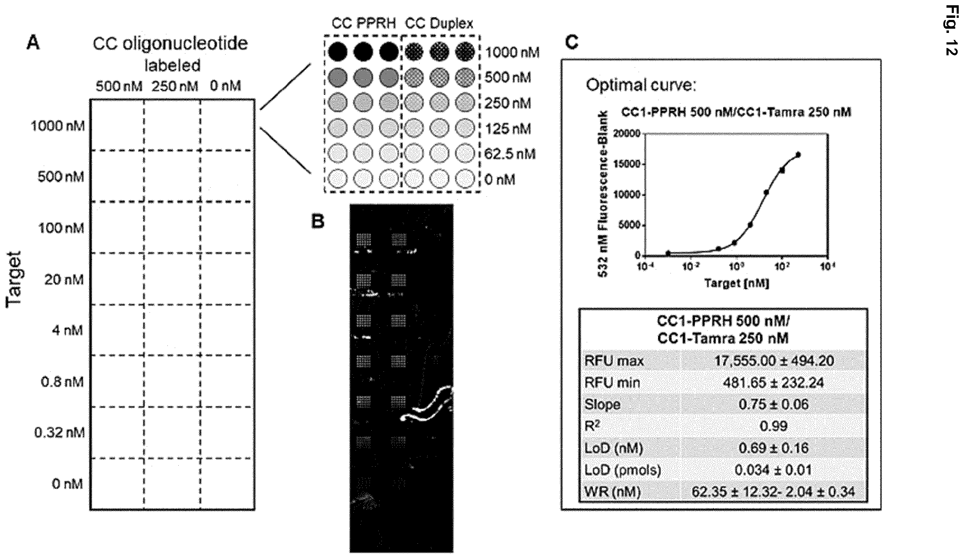

**Fig.12****:** Microarray assay design to test individually each CC oligonucleotide pair using both formats (PPRH and duplex clamp) and results obtained for CC1 pair. **A)** Experimental design of a single oligonucleotide pair microarray standard curve, where different concentrations of the first oligonucleotide in PPRH and duplex format are printed to later on hybridize with the corresponding target also tested at eight different concentrations prepared in hybridization buffer. The detection takes place by using a second oligonucleotide that also hybridizes in a second region of the target and which is labelled with a fluorophore. **B)** Fluorescence image of a microarray assay performed following these scheme (in this case the fluorescence comes from a second oligonucleotide labelled with TAMRA). **C)** Calibration curve obtained testing the CC1 pair and the corresponding analytical parameters of the sigmoidal curve obtained (WR: working range: range over which target concentrations can be quantitated with acceptable precision and reliability).
**Fig. 13****:** Example of a parallel clamp with interruptions in the polypyrimidine target region and in the second polypyrimidine region of the parallel clamp.

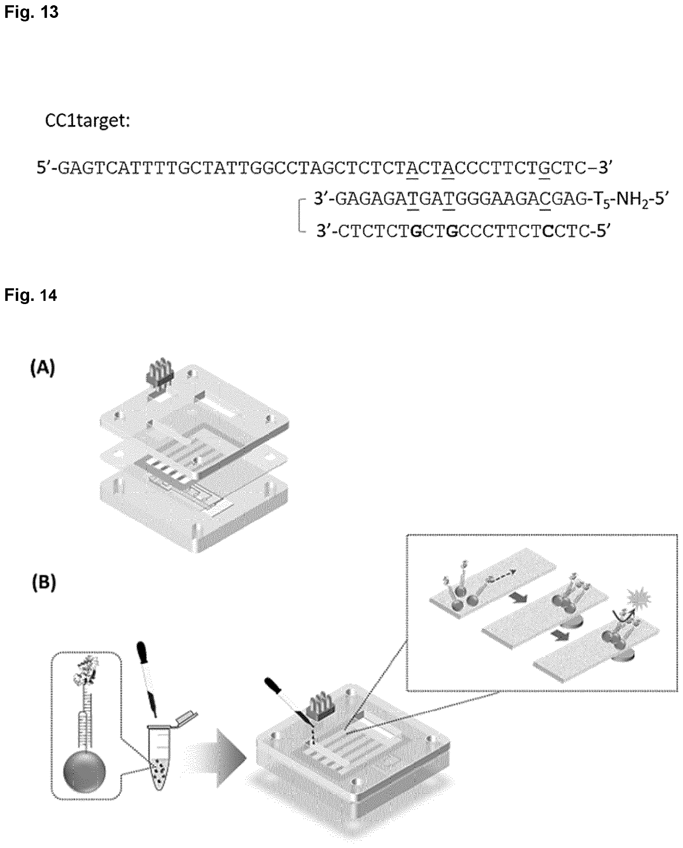

**Fig. 14: (A)** Scheme of the electrochemical system or device prototype showing the bottom and top parts of the cartridge together with the electrochemical transducer array and the paper component sandwiched between them. **(B)** Scheme of the different steps of the electrochemical system or device performance. Left - Sample incubation with magnetic nanoparticles modified with the first oligonucleotide in the presence of the second oligonucleotide conjugated to an enzyme. Middle- Nanoparticle suspension added to the electrochemical device after sample incubation. Right - Steps taken place in the electrochemical device comprising flow of magnetic nanoparticles, accumulation of the magnetic nanoparticles over the magnet inserted in the bottom part of the cartridge and electrochemical detection.
**Fig. 15****:** Electrochemical system or device prototype (left) and chronoamperometric signals recorded for different concentrations of the target RNA sequence using magnetic nanoparticles modified with PPRH CC1 (right).

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "about" when referred to a given amount or quantity is meant to include deviations of plus or minus ten, preferably five, percent.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

As used herein, a "polypurine region" is a region of an oligonucleotide that essentially consists of purines. The nucleotides of purine can be adenine (A) or guanine (G) nucleotides.

The term "5'-end" designates the end of a nucleotide strand that has the fifth carbon in the sugar-ring of the deoxyribose at its terminus. The term "3'-end" designates the end of a nucleotide strand that has the hydroxyl group of the third carbon in the sugar-ring of the deoxyribose at its terminus.

As used herein, a "polypyrimidine target region" is a region of an oligonucleotide that essentially consists of pyrimidines. The pyrimidine nucleotides can be cytosine (C), thymine (T), or uracil (U) nucleotides. Particularly, by "polypyrimidine target region" is referred herein as the polypyrimidine-rich region that must be present in the target polynucleotide, that is, the polynucleotide to be detected by the method presented herein. This "polypyrimidine target region" is the region where the parallel clamps or the PPRHs hybridize due to Watson-Crick base-pairing with the purine strand contained in these clamps or PPRHs.

The term "reverse-Hoogsteen bonds", as used herein, means hydrogen bonds between nucleic acids, preferably purines, with a different binding code than Watson-Crick bonds. For the usage of Hoogsteen structures the presence of polypyrimidine/polypurine stretches is required, as this type of binding can only happen when these sequences are present. Specifically, in reverse-Hoogsteen bonds a purine is bound to another purine identical to the first one, in which the two strands forming reverse-Hoogsteen bonds are in antiparallel orientation (see Fig. 1). Said kind of linkage allows the formation of triplex structures, in which one DNA strand can simultaneously form Watson-Crick bonds with one strand (first polypurine region), and reverse-Hoogsteen bonds with the other (second polypurine region as referred herein) (Praseuth, D. et al. 1999. Biochim Biophys Acta, 1489:181-206). Thus, when binding to DNA/RNA the oligonucleotides of the invention form antiparallel triplexes that need the formation of the triads d(A#A•T) and d(G#G•C) wherein # correspond to reverse-Hoogsteen bonds and • correspond to Watson-Crick bonds, and the orientation between the Watson-Crick polypurine strand and the reverse-Hoogsteen polypurine strand is antiparallel. The main benefit of using reverse-Hoogsteen bonds (or antiparallel Hoogsteen bonds) is that they can be formed at physiological pH, unlike parallel Hoogsteen bonds.

The term "Hoogsteen bonds", as used herein, means hydrogen bonds between nucleic acids, preferably pyrimidines, with a different binding code than Watson-Crick bonds. When binding to DNA/RNA the oligonucleotides of the invention form parallel triplexes that need the formation of the triads d(T#A•T) and d(C⁺#G•C) wherein # correspond to Hoogsteen bonds and • correspond to Watson-Crick bonds, and the orientation between the Watson-Crick polypurine strand and the Hoogsteen polypyrimidine strand is parallel (see Fig. 1). As mentioned above these triplexes are more stable in acid pH because Hoogsteen Cytosine needs to be protonated to form a stable Hoogsteen base pair.

The expression "capable of forming a triplex", as used herein, means that the first oligonucleotide of the invention is able to form a triplex structure with at least one of the strands of the target polynucleotide, wherein the first and second polypurine regions of the first oligonucleotide are linked by reverse-Hoogsteen bonds and the first polypurine region forms Watson-Crick bonds with target polynucleotide strand.

The term "linker molecule" refers to any molecule or compound that can act as a connector between the two regions of the first oligonucleotide. Preferably, the linker molecule is a single-stranded nucleotide sequence, preferably a thymidine-rich sequence.

The term "downstream" refers to a nucleotide sequence that is located 3' to a reference nucleotide sequence or sequences. In certain embodiments, downstream nucleotide sequences relate to sequences that follow the starting point of transcription.

The term "upstream" refers to a nucleotide sequence that is located 5' to a reference nucleotide sequence. In certain embodiments, upstream nucleotide sequences relate to sequences that are located on the 5' region a nucleotide region.

In the context of the present invention "test sample" refers to a representative portion of a material or product to test the method. The material may be solid, liquid, or gas. Preferably, the test sample is a biological sample. In the context of the present invention "biological sample" is understood to be any sample of, relating to, caused by, or affecting life or living organisms, biological processes, such as growth and digestion. Examples of a biological sample may include, but are not limited to cell culture, cell culture supernatant, saliva, tears, urine, blood, serum, plasma, sweat, vaginal fluids, semen, feces, mucous, breast milk, ascites, lymph, pleural effusion, synovial fluid, bone marrow, cerebrospinal fluid, mucous, nasopharyngeal swabs and washings from bodily cavities (e.g., bronchial lavage), hair, tissue, bones, or teeth. Preferably, the biological sample is a liquid sample. Liquid means a state of matter with definite volume but no definite shape at 25 °C, like water.

In the context of the present invention "lateral flow system" or "lateral flow assay" is understood as a system comprising a lateral flow device implementing a methodology to detect, in a specific manner, the presence or absence of a target polynucleotide in a given matrix sample. This type of assay eliminates the need for specialized and costly equipment used in conventional laboratories. Lateral flow technology is used as point of care tool (fast and *in situ),* in a large range of applications, from the widely used home pregnancy test to more specialized tests for clinical, food safety, environmental and industrial applications.

In the context of the present invention "a backing card" is understood as the material where all the membranes (sample, conjugate, membrane and wicking pads) are mounted and connected to each other. At the same time, the backing card provides better stability and handling to the lateral flow device.

In the context of the present invention "a sample pad" is understood as the element of the device where the test sample is introduced or deposited and acts as a sponge that holds the sample fluid. Subsequently, the sample fluid is transported to the membrane by capillary action.

In the context of the present invention "a membrane" is understood as the material (e.g. nitrocellulose) that transports the test sample and provides support to the capture molecules (antibodies, aptamers etc.) and allows or enhances their binding capabilities. Two different capture molecules areas are defined on the membrane, test line area which provides the lateral flow assay result (positive or negative) and control line area which probes the correct performance of the lateral flow assay.

In the context of the present invention "a wicking pad" is understood as element in the device that retains all the assay material acting as a waste container.

In the context of the present invention "DNA microarray chip system" is understood as a system comprising a DNA microarray chip or device that in turn comprises a solid surface onto which DNA molecules have been chemically bonded. Complementary base pairing between the target nucleotide present in a test sample, preferably an isolated biological sample, and the DNA molecules attached on the chip produces light that is measured. In this particular system, the light is produced when a hybridization complex comprising the first oligonucleotide, the second oligonucleotide and the target polynucleotide is formed. Areas on the chip producing light identify the target nucleotide that is present in the test sample. The microarray technology is a platform for the simultaneous analysis of different target sequences in complex fluids, in a high throughput manner. The microarray technology also allows multiplexing (detection of different target polynucleotides at the same time).

In the context of the present invention "electrochemical system" is understood as a system comprising an electrochemical device that provides an analytical signal in the form of an electrical current, which is directly proportional to the concentration of an analyte or, in this case, a target polynucleotide. Thus, the electrochemical system or device may act as biosensor. Detection of the at least one target polynucleotide is achieved due to production of a faradaic current produced due to the binding of the target polynucleotide to the first and second oligonucleotides according to the present invention. The second oligonucleotide is conjugated to a reported molecule, an enzyme, whose reaction with a suitable substrate, produces an electroactive chemical compound. The redox process of the produced electroactive compound taking place on the electrochemical device produces a current upon application of a voltage that is directly proportional to the concentration of the target polynucleotide.

### DESCRIPTION OF THE EMBODIMENTS

There is a constant need for quick and cheap methods that allow the detection of polynucleotides, such as pathogen genomes, in a sensitive, efficient but also low-cost way. In many cases, rapid diagnostic tests lack of enough sensitivity, and highly sensitive tests require of specialized techniques that are expensive and time consuming. For example, in the case of SARS-CoV-2 genome detection and determination of viral load, the current gold standard method to detect infection with high accuracy is the real time reverse-transcriptase polymerase chain reaction (RT-PCR). However, RT-PCR methods require laboratory management of the sample, specialized professionals and several hours until the result is obtained.

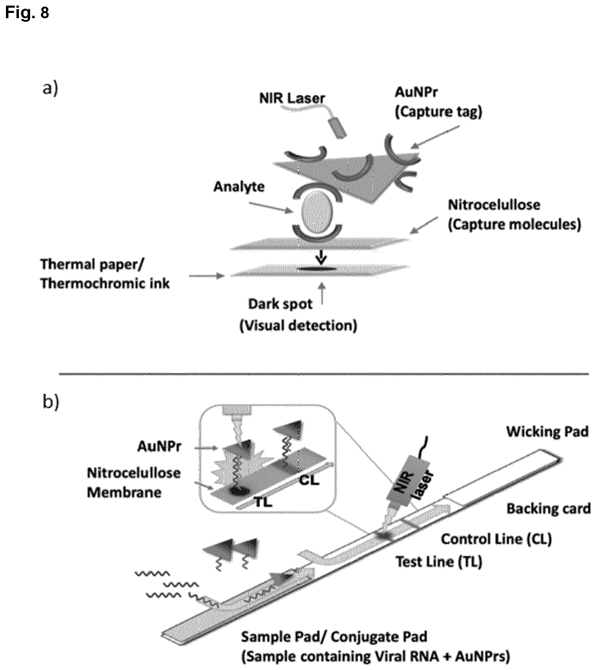

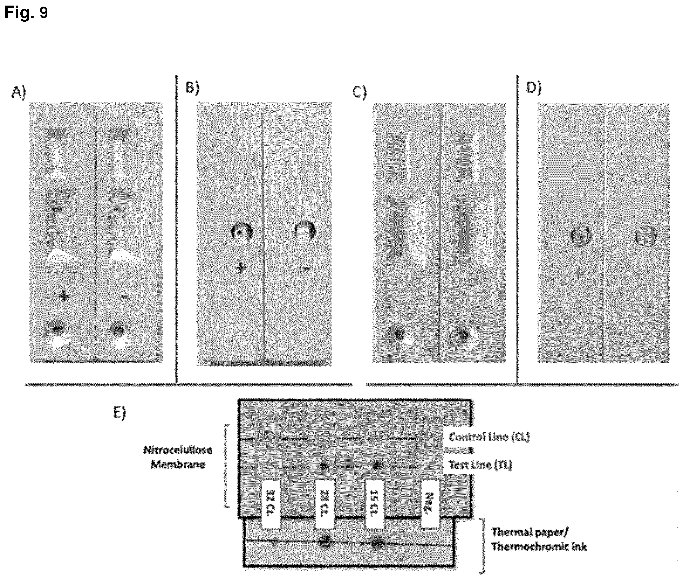

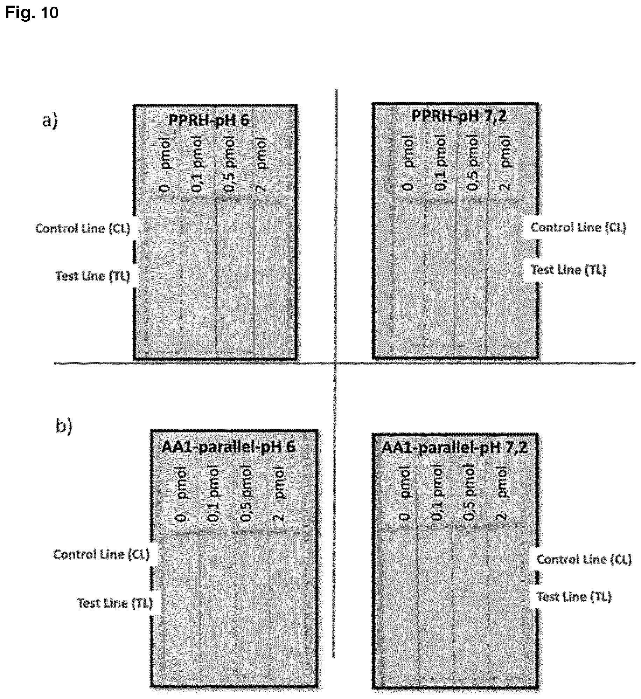

The present invention provides an efficient and sensitive method based on the use of PPRHs or parallel clamps in combination with other oligonucleotides to detect target polynucleotides. Further, the method provided herein can be implemented in lateral flow assays, microarrays and multiplexed electrochemical devices without compromising its efficiency and sensitivity and be used in point of care or laboratory settings. As shown in Fig. 10A and B, both the PPRH and parallel clamps oligonucleotides (also called herein first oligonucleotides) in combination with another oligonucleotide (also called herein second oligonucleotide) can be used to detect up to 0.1 pmol (for PPRHs) and 0.5 pmol (parallel clamps) of target polynucleotide in a Lateral Flow assay (see Fig. 8A and B for a scheme of a Thermo-lateral flow system or device). Further, clinical samples obtained from nasopharyngeal swab of patients infected with SARS-CoV-2 virus were assayed to determine the viral load present in said samples in a comparative analysis using, on the one hand, the gold standard technique (RT-PCR) and, on the other hand, a thermo lateral flow assay using the method of the present invention. The results obtained surprisingly show that a thermo lateral flow system using the method of the present invention was able to detect SARS-CoV-2 viral genome at a sensitivity comparable of up to 32 Ct as determined by qPCR (see Fig. 9), demonstrating that the method provided herein is highly sensitive.

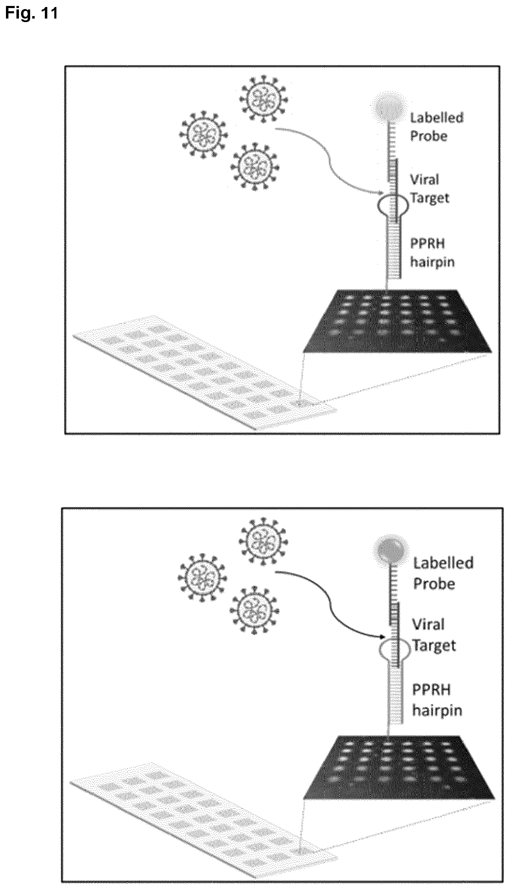

On the other hand, the microarray chip system or device shown in Fig 11 has been used to simultaneously detect distinct target polynucleotide sequences of the virus in the commonly employed Universal Transport Media (UTM) providing in this way improved specificity to the method. The sensitivity achieved is in the same order of magnitude as in the thermo lateral flow assay system and allows quantification of the viral load, a parameter of great interests for diagnostics, if implemented on a clinical laboratory. Moreover, the microarray chip system or device has high sample throughput capabilities allowing to process simultaneously more than 100 test samples in less than 30 min. Moreover, the microarray chip system or device using this method presents great potential for differentiating between the different virus variants in the same sample run.

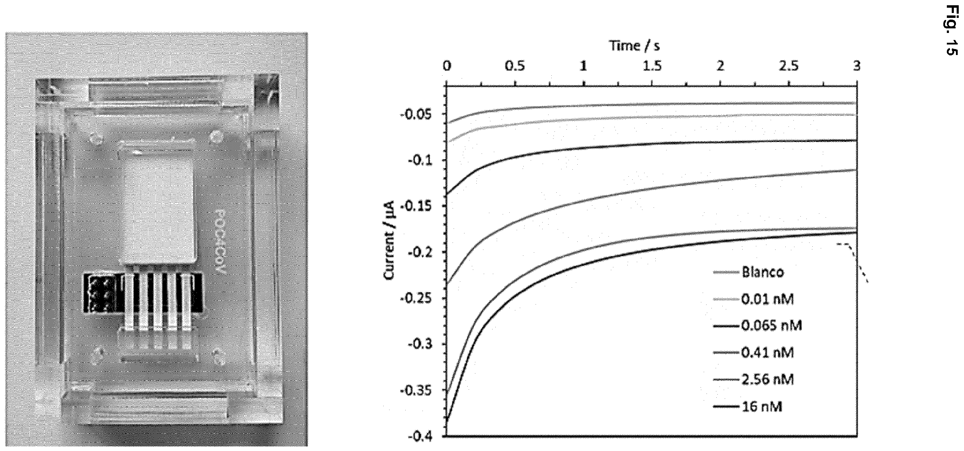

Moreover, the multiplexed electrochemical system or device showed in Fig. 14 has been applied to the quantitative simultaneous detection of 3 to 5 polynucleotide sequences of the viral RNA in buffer and several universal transport media, showing the applicability of the device where the method has been implemented as a point-of-care diagnostic tool for efficiently and rapidly detecting the virus and quantitatively estimating the viral load. The sensitivity and limits of detection are of the same order of magnitude to those shown by the lateral flow assay and the microarray (see Fig. 9 (LFA), 12 (microarray) Fig. 15 (electrochemical device)), demonstrating that the method provided herein represents a solid method with multiple applications.

In conclusion, the method of the present invention represents a tool to detect in a fast, low cost, sensitive and efficient way even small amounts of a target polynucleotide in a point of care or laboratory setting, without the need of using expensive, laboratory-based and time consuming techniques and without compromising the sensitivity and accuracy of the detection.

Thus, in a **first aspect,** the invention relates to an *in vitro* method for detecting at least one target polynucleotide in a test sample, preferably an isolated biological sample, comprising the steps of:
i) contacting the test sample with a first oligonucleotide, wherein the first oligonucleotide comprises a first polypurine region and a second region, wherein said first and second regions are connected by a linker molecule, and wherein:
   a) the second region is a polypurine region and said first and second regions are capable of forming a hairpin maintained by reverse-Hoogsteen bonds with the two strands running in antiparallel orientation, or
   b) the second region is a polypyrimidine region and said first and second regions are capable of forming a hairpin maintained by Hoogsteen bonds with the two strands running in parallel orientation;

   wherein the first region is of at least 12 nucleotides in length and wherein at least 6 nucleotides of the at least 12 nucleotides of the first region are engaged forming said hairpin with the second region; and
   wherein the first region is substantially complementary to a polypyrimidine target region comprised in the at least one target polynucleotide, wherein the hybridization between the first polypurine region and the polypyrimidine target region forms a structure comprising a triplex; and
ii) contacting the test sample with a second oligonucleotide, wherein the second oligonucleotide is substantially complementary to the at least one target polynucleotide; and
iii) detecting the hybridization between the first and the second oligonucleotides to the at least one target polynucleotide.

In an embodiment, the at least one target polynucleotide is the genome of at least an infectious agent, including bacteria, fungi, viruses, and parasites. In a preferred embodiment, the at least one target polynucleotide detected is one or more viral genomes or viral polynucleotides. In a further preferred embodiment, the viral polynucleotide is single-stranded, preferably RNA single-stranded. In a preferred embodiment, the viral polynucleotide is selected from the group consisting of coronavirus, Influenza virus, and/or Respiratory syncytial virus (RSV) viral genomes, or a combination thereof. Preferably, the coronavirus is Severe acute respiratory syndrome coronavirus 2 (SARS-CoV2). Preferably, the Influenza virus is H1N1 strain. Preferably, the RSV is RSV B strain. In an embodiment, two or more than two different target polynucleotides (i.e., with different polynucleotide sequences) comprised in test sample are detected.

In steps i) and ii) of the *in vitro* method according to the first aspect, a first and a second oligonucleotide are used, respectively. The first oligonucleotide can be a polypurine reverse-Hoogsteen hairpin (PPRH) that comprises a first and a second polypurine regions physically linked to each other by a linker molecule, preferably a single-stranded nucleotide region. Alternatively, the first oligonucleotide can be a parallel clamp that comprises a first polypurine region and a second polypyrimidine region physically linked to each other by a linker molecule, preferably a single-stranded nucleotide region. In both cases (PPRH and parallel clamp), the first polypurine is substantially complementary to a polypyrimidine target region comprised in the at least one target polynucleotide, wherein the hybridization between the first polypurine region and the polypyrimidine target region forms a structure comprising a triplex.

The second oligonucleotide used in step ii) is a single-stranded oligonucleotide that hybridizes with the at least one target polynucleotide. Each of these oligonucleotides are described in detail below together with the steps where they are used.

Step i) of the first aspect comprises contacting the test sample, which is preferably a biological sample, with the first oligonucleotide. In an embodiment, the "first oligonucleotide" is a polypurine reverse-Hoogsteen hairpin (hereinafter referred to as PPRH). In an embodiment, the "first oligonucleotide" is a parallel clamp.

In the context of the present invention, "polypurine reverse-Hoogsteen hairpin or PPRH" means a first oligonucleotide that comprises or consists of a first polypurine region or strand and a second polypurine region or strand, wherein said first and second polypurine regions are running in antiparallel orientation and form reverse-Hoogsteen bonds between them. By "antiparallel orientation" is referred herein as the two strands of the helix run in opposite directions, so that the 5' carbon end of one strand faces the 3' carbon end of its matching strand. Said first and second polypurine regions are further physically connected by a linker molecule, preferably a single-stranded nucleotide region, forming a hairpin.

In the context of the present invention, by "parallel clamp" is meant a first oligonucleotide that comprises or consists of a first polypurine region similar to the first polypurine region described above for PPRH, and a second polypyrimidine region, wherein said first polypurine region and said second polypurine region are running in parallel orientation and form Hoogsteen bonds between them. By "parallel orientation" is referred herein as the two strands of the helix run in the same direction, so that the 5' carbon end of one strand faces the 5' carbon end of its matching strand. Similar as for the PPRH, the first and second regions of the parallel clamp are further physically connected by a linker molecule, preferably a single-stranded nucleotide region, forming a hairpin.

It is noted that both the PPRH and the parallel clamp have in common that they comprise a first polypurine region. In the present invention, we designate as "first polypurine region" the polypurine region of the first oligonucleotide that forms Watson-Crick bonds with a polypyrimidine target region located in the at least one target polynucleotide. Further, we designate as "second region" the region of the first oligonucleotide that binds by reverse-Hoogsteen bonds (if the second region is a polypurine region, such as in PPRH) or by Hoogsteen bonds (if the second region is a polypyrimidine region, such as in parallel clamps) to the first polypurine region. Thus, the terms "first polypurine region" and "second region" are defined herein on the basis of which region they bind, but these terms do not refer to the order in which said polypurine regions are found in the linear first oligonucleotide. The terms "first polypurine region", "second region" and "polypyrimidine target region" are further characterized in detail below:
"First polypurine region", as used herein, means the motif comprised in the first oligonucleotide comprising a succession of at least 12 purine nucleotides that is, on the one hand, able to hybridize by means of Watson-Crick bonds with the polypyrimidine target region and, on the other hand, able to bind by reverse-Hoogsteen bonds or by Hoogsteen bonds with the second region.

In an embodiment, the first polypurine region comprised in the first oligonucleotide is at least 12, 13, 14, 15, 16, 17, 18, 19, 20, 21, 22, 23, 24, 25, 30, or 35 nucleotides in length. In another embodiment, the first polypurine region has a length ranging from 12-20, 12-25, 15-25, 20-25, 20-30, 20-35 nucleotides or any value in between. Preferably, the first polypurine region is about 20 nucleotides in length. Also, preferably, the first polypurine region consists of a succession of consecutive purines.

By "second polypurine region", as used herein, is meant the motif comprised in the PPRH (first oligonucleotide) comprising or consisting of a succession of at least 6 purine nucleotides that form reverse-Hoogsteen bonds with the first polypurine region. When the target polypyrimidine strand contains a purine interruption, the second polypurine region is identical to the first polypurine region but in antiparallel orientation. Although a reverse-Hoogsteen bond (such as d(T#T•A) or d(C#C•G) triads) cannot be formed, the experience has demonstrated that this configuration allows to overcome the presence of a few purine interruptions in the polypyrimidine targets. This situation has been defined as mirror repeat sequence to the first polypurine region. Thus, in the context of the present invention, "mirror repeat sequence" refers to repeats that are inverse exact (i.e. not complementary) repeats and may form part of triple helices in DNA carrying a one, two, three, or more than three interruptions in the polypyrimidine track. Thus, when the first and the second polypurine regions are mirror repeat sequences they are delimited on the basis of them containing a center of symmetry on a single strand. In an embodiment, the second polypurine region comprised in the first oligonucleotide is at least 6, 7, 8, 9, 10, 11,12, 13, 14, 15, 16, 17, 18, 19, 20, 21, 22, 23, 24, 25, 30, or 35 nucleotides in length. In another embodiment, the second polypurine region has a length ranging from 6-20, 12-20, 12-25, 15-25, 20-25, 20-30, 20-35 nucleotides or any value in between. Preferably, the second polypurine region is about 20 nucleotides in length. Also, preferably, the second polypurine region consists of a succession of consecutive purines.

By "second polypyrimidine region", as used herein, is meant the motif comprised in the parallel clamps (first oligonucleotide) consisting of or comprising a succession of at least 6 pyrimidines nucleotides that form Hoogsteen bonds with the first polypurine region. When the target polypyrimidine strand contains a purine interruption then the second polypyrimidine region will contain a G when adenine is the interruption and a C when guanine is the interruption, Although a Hoogsteen bond (such as d(G#T•A) or d(C#C•G) triads, Fig. 13) cannot be formed, the experience has demonstrated that this configuration allows to overcome the presence of a few purine interruptions in the polypyrimidine targets. In an embodiment, the second polypyrimidine region comprised in the first oligonucleotide is at least 6, 7, 8, 9, 10, 11,12, 13, 14, 15, 16, 17, 18, 19, 20, 21, 22, 23, 24, 25, 30, or 35 nucleotides in length. In another embodiment, the second polypyrimidine region has a length ranging from 6-20, 12-20, 12-25, 15-25, 20-25, 20-30, 20-35 nucleotides or any value in between. Preferably, the second polypyrimidine region is about 20 nucleotides in length. Also, preferably, the second polypyrimidine region consists of a succession of consecutive pyrimidines.

It is noted that in the present invention, when we refer to "second region" in relation to the first oligonucleotide we refer to both the "second polypurine region" (which is present in PPRH), and the "second polypyrimidine region" (which is present in the parallel clamps).

Preferably, the first oligonucleotide is a PPRH, that is, it is preferred that the second region of the first oligonucleotide is a polypurine region and that the first and second polypurine regions are capable of forming a hairpin maintained by reverse-Hoogsteen bonds with the two strands running in antiparallel orientation.

In an embodiment, the first oligonucleotide consists of a nucleic acid polymer composed of non-modified oligodeoxyribonucleotides. In an embodiment, the first oligonucleotide consists or comprises of a first polypurine region and a second region. In a preferred embodiment, the total length of the first oligonucleotide is at least 21, 30, 35, 40, 45, 50, 60, 70, 80, 90, or 100 nucleotides. In a preferred embodiment, the total length of the first oligonucleotide is between 21 to 100, 30 to 100, 40 to 100, or 50 to 100 nucleotides.

As stated above, the first polypurine region and the second region are capable of forming a hairpin. If the first oligonucleotide is a PPRH, the second region is a polypurine region and therefore the first polypurine region and the second polypurine region form an antiparallel hairpin wherein both regions run in an antiparallel orientation. The term "antiparallel hairpin", as used herein, refers to a stable duplex formed by the PPRH molecule that doubles back on itself to form a two stranded structure maintained by intramolecular reverse-Hoogsteen bonds between the first and second polypurine regions comprised in the PPRH. In an embodiment, the sequence of the second polypurine region is a mirror repeat of the sequence of the first polypurine region.

If the first oligonucleotide is a parallel clamp, the second region is a polypyrimidine region and therefore the first polypurine region and the second polypyrimidine region form a parallel hairpin wherein both regions run in a parallel orientation. The term "parallel hairpin", as used herein, refers to a stable duplex formed by the parallel clamp molecule that doubles back on itself to form a two stranded structure maintained by intramolecular Hoogsteen bonds between the first polypurine region and the second polypyrimidine region comprised in the parallel clamp.

In some embodiments, the nucleotides of the first oligonucleotide are not modified. In some other embodiments, the nucleotides of the first oligonucleotide may be modified to stabilize the hairpin. In an embodiment, the modifications include, but are not limited to, amine modified nucleotides, such as 8-aminopurines such as 8-amino-2'-deoxyguanine and/or 8-amino-2'-deoxyadenine modifications. Most of the times the PPRHs do not necessarily comprise 8-aminopurine modifications and they can be easily obtained from commercial sources. On the other hand, it has been described that 8-amino-2'-deoxyguanine stabilizes both parallel (Soliva 2000) and antiparallel (Aviñó 2003) triplexes but 8-amino-2'-deoxyadenosine stabilizes only parallel triplexes (Güimil-García 1999, Aviñó, 2003). Thus, in some embodiments, the parallel clamps may comprise 8-aminoguanine and/or 8 aminoadenine residues and/or their correspondent deoxy-derivatives (i.e., 8-amino-2'-deoxyguanine and/or 8-amino-2'-deoxyadenosine) in the first polypurine region. Further, in some other embodiments, the PPRH may comprise 8-aminoguanine, and/or 8-amino-2'-deoxyguanine, but not 8-amino-2'-deoxyadenosine residues in the first polypurine region.

It is noted that, while the minimum length of the first polypurine region is at least 12 nucleotides, the minimum length of the second region is of at least 6 nucleotides. Thus, the hairpin formed in the first oligonucleotide can be symmetric or asymmetric, that is, the first polypurine region and the second region may be of the same length, forming a symmetric hairpin; or they may have different lengths, forming an asymmetric hairpin. In asymmetric hairpins where the second region is shorter that the first polypurine region, not all the purines from the first polypurine region will be engaged in the reverse-Hoogsteen (PPRH) or Hoogsteen (parallel) bonds with the nucleotides of the second region. In a preferred embodiment, the first polypurine region comprises or consists of at least 12 nucleotides in length of which at least 6 nucleotides are engaged forming the hairpin with the nucleotides of the second polypurine region. In a preferred embodiment, the first polypurine region and the second region comprised in the first oligonucleotide are identical in length and thus they form a symmetrical hairpin where all the purines of the first polypurine region are engaged in the hairpin with the nucleotides of the second region. Thus, in a possible embodiment, all the nucleotides of the first polypurine region and the second region are engaged in bonds, particularly in reverse-Hoogsteen (in case of PPRH) or Hoogsteen (in case of parallel clamp) bonds.

As stated above, the first polypurine region and the second region of the first oligonucleotide are also physically connected by a linker molecule. In a preferred embodiment, the linker molecule is a single-stranded region. In a preferred embodiment, the single-stranded region is a short region of one, two, three, four, five, six, seven, eight, nine, ten or more nucleotides. Preferably, the single-stranded region that connects the first polypurine region and the second region is a thymidine-rich region. By "thymidine-rich region", as used herein, is meant a single-stranded sequence, formed exclusively by thymidine (T) nucleotides, that directly connects the two regions of the first oligonucleotide. In an embodiment, there are no other nucleotides between the first and the second region and the thymidine-rich region. In an embodiment, the minimum number of thymidines nucleotides in said region is three, preferably four. In another embodiment, the length of the thymidine-rich region may vary between 3 and 12 thymidines, preferably between 4 and 10 thymidines, more preferably between 4 and 8 thymidines, most preferably between 3 and 5 thymidines. In a preferred embodiment, the thymidine-rich region consists of between 3 to 5 thymidines. In a preferred embodiment of the first aspect of the present invention, the thymidine-rich region is a four-thymidine region.

The single-stranded region that connects the first polypurine region with the second region and that is preferably a thymidine-rich region forms a loop when the first oligonucleotide of the invention is in the form of a duplex or hairpin, that is, when the first and the second regions comprised in the first oligonucleotide bind each other by means of reverse-Hoogsteen (PPRH) or Hoogsteen (parallel clamps) bonds. As used herein, "duplex structure" or "hairpin" refer to the same structure and are used interchangeably. Said loop may be on either side of the hairpin. In a preferred embodiment, in the case of PPRH, the single-stranded nucleotide region, preferably the thymidine-rich region, comprised in the first oligonucleotide connects the 3'-end of the first polypurine region and the 5'-end of the second polypurine region or the single-stranded nucleotide region connects the 5'-end of the first polypurine region and the 3'-end of the second polypurine region. In the case of parallel clamps, the single-stranded nucleotide region, preferably the thymidine-rich region, comprised in the first oligonucleotide connects the 3'-end of the first polypyrimidine region and the 5'-end of the second polypyrimidine region or the single-stranded nucleotide region connects the 5'-end of the first polypurine region and the 3'-end of the second polypyrimidine region.

As an alternative to the single-stranded thymidine-rich region, other compounds can serve as linkers molecules between the first and the second region of the first oligonucleotide including any nucleotide sequence and in special the GGAGG and CTTTG described in Aviñó et al 2002 (Aviñó et al 2002, "Properties of triple helices formed by parallel-stranded hairpins containing 8-aminopurines." Nucleic Acids Res., 30, 2609-2619). In another embodiment, the linker molecule is an organic linker. In a preferred embodiment, the organic linker is hexaethyleneglycol (HEG) or similar thereof. In a preferred embodiment, preferably in the case of parallel clamps, the linker molecule, preferably the organic linker, comprised in the first oligonucleotide connects the 3'-end of the first polypurine region and the 3-end of the second region; or the linker molecule, preferably organic linker, connects the 5'-end of the first polypurine region and the 5'-end of the second region.

As stated above, the first polypurine region hybridizes by means of Watson-Crick bonds with a polypyrimidine target region. The polypyrimidine target region is comprised in the at least one target polynucleotide to be detected by the method according to the first aspect or any of its embodiments. As defined above, the polypyrimidine target region is a motif rich in pyrimidines defined by its ability to hybridize by means of Watson-Crick bonds with the first polypurine region of the first oligonucleotide.

Thus, in an embodiment, the first polypurine region is substantially complementary to the polypyrimidine target region. By "substantially complementary" with respect to the first polypurine region of the first oligonucleotide in relation to the polypyrimidine target region is meant a polypurine region having a percentage of complementarity between the substantially complementary polypyrimidine target region and the exact complementary first polypurine region of at least 60%, at least 70%, at least 80%, at least 85%, at least 90%, at least 93%, at least 95%, at least 96%, at least 97%, at least 98%, at least 99% or at least 100%. Identity is assessed over the entire length of the polypyrimidine region to the polypurine region. The degree of identity between two nucleotide regions can be determined by the skilled artisan, for instance by using BLAS-TN algorithm (BLAST Manual, Altschul, S. et al., NCBI NLM NIH Bethesda, Md. 20894, Altschul, S., et al., J., 1990, Mol. Biol. 215:403-410). A polypyrimidine region "substantially complementary" to the first polypurine region hybridizes to the polypurine region under low stringency conditions, preferably medium stringency conditions, most preferably high stringency conditions.

In a preferred embodiment, the first polypurine region comprised in the first oligonucleotide is at least 70%, 80%, 90%, 92%, 95%, 96%, 97%, 98%, 99% or, preferably, 100% complementary, over its full length, to the polypyrimidine target region comprised in the at least one target polynucleotide.

The polypyrimidine target region comprises a succession of pyrimidines. In a preferred embodiment, said succession consists of a consecutive succession of only pyrimidines, that is, a succession of pure pyrimidines. When there are no pure polypyrimidine regions in the at least one target polynucleotide and said regions have one or more purine interruptions, it is still possible to design the first oligonucleotide of the present invention by placing the corresponding complementary pyrimidine in the first polypurine region.

Thus, in a preferred embodiment, at the time of designing the first polypurine region of a PPRH, and if there are purine interruptions in the polypyrimidine target region, the complementary base by Watson:Crick rules (that is, the complementary pyrimidine) can be introduced in the first polypurine region, so that the first polypurine region and the polypyrimidine target region are 100% complementary, without the presence of non-complementary nucleotides, thereby they hybridize completely.

Further, when the first oligonucleotide is a parallel clamp, if purine interruptions are present in the polypyrimidine target region and their corresponding complementary pyrimidine is placed in the first polypurine region to achieve 100% complementary between both strands, the following rule can optionally be applied:
- If there is a "T" in the first polypurine region of the parallel clamp, and therefore there is an "A" nucleotide in the corresponding position in the polypyrimidine target region, a "G" is preferably placed (rather than a "A") in the corresponding position of the second polypyrimidine region of the parallel clamp (see Fig. 13, wherein the "G" is highlighted in bold and the "T" and "A" are underlined).
- If there is a "C" in the first polypurine region of the parallel clamp, and therefore there is a "G" nucleotide in the corresponding position in the polypyrimidine target region, a "C" is preferably placed (rather than a "G") in the corresponding position of the second polypyrimidine region of the parallel clamp (see Fig. 13, wherein the "C" is highlighted in bold and the "T" and "A" are underlined).

These modifications are advantageous as described in Aviñó et 2002 (Aviñó et al 2002, "Properties of triple helices formed by parallel-stranded hairpins containing 8-aminopurines." Nucleic Acids Res., 30, 2609-2619).

In an alternative embodiment, the polypyrimidine target region comprises a succession of pyrimidines with one, two, three or more purine interruptions. In an embodiment, a maximum of three purine interruptions in the polypyrimidine target region is allowed.

In an embodiment, the polypyrimidine target region has between 12 nucleotides and the maximum possible length with the first polypurine region, preferably at least 20 nucleotides, more preferably at least 24 nucleotides, most preferably at least 30 nucleotides. When the at least one target polynucleotide to be detected is DNA, the nucleotides of the polypyrimidine target region can be cytosine (C) or thymine (T) nucleotides. When the at least one target polynucleotide RNA the nucleotides of the polypyrimidine target region can be cytosine (C) or uracil (U) nucleotides.

Both the first and the second oligonucleotides of the invention must bind specifically to the polypyrimidine target region. As it is used herein, the expression "binds specifically to" refers to the capacity of the oligonucleotides for binding specifically to their target sequence and not to other sequence. The specificity may be validated for instance by performing BLAST analysis.

In a preferred embodiment in which the first oligonucleotide is a PPRH, all the purines in the first polypurine region and all the purines in the second region are bound with reverse-Hoogsteen bonds. However, although it is preferred that the first and the second polypurine regions comprise a succession of only purine nucleotides with no polypyrimidine interruptions (consecutive succession of only purines), one, two, three, four, or more polypyrimidine interruptions in the first and the second polypurine regions may be present. In an embodiment, a maximum of one, two, or three pyrimidine interruptions are present in the first region, in the second region, or in both, so the reverse-Hoogsteen bonds formed between the first and the second polypurine regions of the PPRH may not be formed throughout the whole hairpin. In another embodiment, more than three pyrimidine interruptions may be present between the first and the second polypurine regions as long as the hairpin of the first oligonucleotide maintains its duplex structure and it is able to form the triplex structure with the at least one target polynucleotide, particularly with the polypyrimidine target region.

In a preferred embodiment in which the first oligonucleotide is a parallel clamp, all the purines in the first polypurine region and all the pyrimidines in the second region are bound with Hoogsteen bonds. However, although it is preferred that the first and the second regions comprise a succession of only purine/pyrimidine nucleotides with no interruptions, one, two, three, four, or more interruptions in said regions may be present. In an embodiment, a maximum of one, two, or three interruptions are present in the first region, in the second region, or in both, so the Hoogsteen bonds formed between the first and the second regions of the parallel clamp may not be formed throughout the whole hairpin. In another embodiment, more than three interruptions may be present between the first and the second regions as long as the hairpin of the first oligonucleotide maintains its duplex structure and it is able to form the triplex structure with the at least one target polynucleotide, particularly with the polypyrimidine target region.

In an embodiment, the first oligonucleotide comprises, consists, or consists essentially of a sequence selected from the group consisting of SEQ ID NOs: 60, 62, 63, 64, 67, 68, 65, or 66, or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 60, 62, 63, 64, 67, 68, 65, or 66.

The design of the oligonucleotides of the invention for a determined target polynucleotide is performed using a software tool to search for polypurine stretches and their complementary polypyrimidine sequences in the DNA sequence of the at least one target polynucleotide. A suitable software can be the Triplex-Forming Oligonucleotide Target Sequence Search software (M.D. Anderson Cancer Center, Houston, TX) (spi.mdanderson.org/tfo/). Even in the case of the presence of purine interruptions in the polypyrimidine target sequence, PPRHs and parallel clamps against that sequence can be designed and used efficiently by introducing the complementary base to the purine interruption in the first polypurine region in the position in front of the interruption.

In an embodiment, the first oligonucleotide is a PPRH and it is synthesized in the form of linear oligonucleotide. The synthesis of the first oligonucleotide may be performed according to conventional methods known in the state of the art (Methods in Molecular Biology, vol. 288, "Oligonucleotide synthesis: Methods and Applications". Piet Herdewijn, Ed., Humana Press, 2005). Thus, a skilled person would know how to design and synthetize the first oligonucleotide of the present invention considering the features described above. As a mode of example, a standard protocol for the design of PPRHs is provided herein in Anna Aviñó et al. 2019. doi: 10.1002/cpnc.78 (see Basic protocol 1). The protocol to design the first oligonucleotide can also be found in the Examples of the present invention. Briefly, polypurine stretches are found in the at least one target polynucleotide and the complementary sequence of each of these regions constitutes the polypyrimidine target region that will be targeted by the PPRH. Then, the selection of sequences to build the PPRHs, among all the possible matches found in the search, is made considering the length of the sequence (the longer the sequence is, the more specific the hybridization will be), the number of pyrimidine interruptions (preferably, a maximum of 3) and the specificity of that sequence within the at least one target polynucleotide (whether there are similar regions in the at least one target polynucleotide that may compete for the binding of the designed PPRHs). The first polypurine region will be designed considering the selected polypyrimidine target region, and then, the second polypurine region is selected by mirror repeating its sequence, that is by determining the reverse sequence of the first polypurine region. Then, the final PPRH sequence is assembled by connecting the first and the second polypurine regions using a thymidine rich single stranded region spaced, which will produce the loop to allow the formation of the hairpin. Non-limiting examples of software tools that may be used for the design of the PPRH are: Triplex-Forming Oligonucleotide Target Sequence Search software (available at http://utw10685.utweb.utexas.edu/tfo/), Triplex target site Mapping and Integration software (available at http://ttsmi.bii.a-star.edu.sg); Reverse Complement software (available at https://www.bioinformatics.org/sms/rev_comp.html), BLASTn software (available at https://blast.ncbi.nlm.nih.gov/Blast.cgi?PAGE_TYPE=BlastSearch).

In an alternative embodiment, the first oligonucleotide is a parallel clamp and it is synthesized as also described in Anna Aviñó et al. 2019. doi: 10.1002/cpnc.78. The protocol to design the first oligonucleotide can also be found in the Examples of the present invention. Briefly, the preparation of 5'-5' hairpins (parallel clamps in C configuration) is performed in three parts: first, the first polypurine region is synthetized and assembled. If the first polypurine region comprises modified 8-aminopurines, they are synthetized using standard phosphoramidites for the natural bases and the 8-aminopurine phosphoramidites. Methods to obtain 8-aminoadenine or 8-aminoguanine are known by the skilled person. Once the first polypurine region is obtained, a hexaethyleneglycol linker or a tetra- or pentathymidine sequence is added using the appropriate phosphoramidite. Alternatively, both regions of the parallel clamp are linked by a single-stranded nucleotide region or sequence. Finally, the pyrimidine part is assembled using reversed C and T phosphoramidites. For the preparation of 3'-3' hairpins (D configuration) a similar approach is used. In an embodiment, the second pyrimidine region may be assembled on sold support functionalized with reversed C or reversed T and reversed phosphoramidites, and then the phosphoramidites hexaethyleneglycol or a tetra- or pentathymidine sequence is added. Alternatively, both regions of the parallel clamp are linked by a single-stranded nucleotide region or sequence. Similar software tools as described for PPRH design can be used for parallel clamp design. Alternatively the pyrimidine strand can carry any of the nucleoside derivatives that have been described to stabilize the triplex such as 5-methyl-C, 5-bromo-U, 5-propynyl-C, 5-propynyl-U, pseudo-U, pseudoiso-C, pyrido[2,3-d]pyrimidine, benzo[f]quinazoline, 6-oxo-C, 6-amino-C, 2-aminopyridine, 8-oxo-A, N7-alkyl-G reviewed in chapter 5 of the book Nucleic Acids Chemistry. Modifications and conjugates for biomedicine and nanotechnology edited by Eritja (2021, de Gruyter Ed).

Step ii) of the first aspect comprises contacting the test sample, which is preferably an isolated biological sample, with the second oligonucleotide. As used herein, the "second oligonucleotide" is a single-stranded nucleotide polymer that is independent of the first oligonucleotide and that is designed to hybridize with the target polynucleotide. The second oligonucleotide comprises at least 12, 20, 25, 30, or 35 nucleotides in length and is substantially complementary, preferably about 85%, 90%, 95%, 96%, 97%, 98%, 99%, or 100% complementary to the target polynucleotide.

In an embodiment, the second oligonucleotide is substantially complementary to the target polynucleotide. Preferably, the second oligonucleotide is substantially complementary to a region of the target polynucleotide. It is noted that the hybridization of the second oligonucleotide should not interfere with the hybridization of the first oligonucleotide with the polypyrimidine target region. Thus, in an embodiment, the second oligonucleotide does not hybridize in the same region of the target polynucleotide as the first polypurine region. In an embodiment, the second oligonucleotide hybridizes at least 1, 2, 3, 4, 5, 10, 15, 20, 25, or 30 nucleotides upstream or downstream (that is, before or after) of the hybridization region of the first oligonucleotide.

In an embodiment, the second oligonucleotide comprises, consists, or consists essentially of a sequence selected from the group consisting of SEQ ID NOs: 5, 12, 13, 19, 25, 26, 33, or 34 or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 5, 12, 13, 19, 25, 26, 33 or 34.

In an embodiment of the *in vitro* method according to the first aspect or any of its embodiments, the at least one target polynucleotide is from a coronavirus, preferably a SARS coronavirus, more preferably SARS-CoV-2 virus, and the first and the second oligonucleotides comprise, consist or consist essentially of a sequence selected from the list consisting of:
a) SEQ ID NOs: 62 (first oligonucleotide) and 5 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 62 and 5,
b) SEQ ID NOs: 60 (first oligonucleotide) and 5 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 60 and 5,
c) SEQ ID NOs: 63 (first oligonucleotide) and 12 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 63 and 12,
d) SEQ ID NOs: 63 (first oligonucleotide) and 13 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 63 and 13, or
e) SEQ ID NOs: 64 (first oligonucleotide) and 19 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 64 and 19.

In an embodiment of the *in vitro* method according to the first aspect or any of its embodiments, the at least one target polynucleotide is from Influenza virus, preferably Influenza H1N1 virus, and the first and the second oligonucleotides comprise, consist or consist essentially of a sequence selected from the list consisting of:
a) SEQ ID NOs: 67 (first oligonucleotide) and 33 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 67 and 33, or
b) SEQ ID NOs: 68 (first oligonucleotide) and 34 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 68 and 34.

In an embodiment of the *in vitro* method according to the first aspect or any of its embodiments, the at least one target polynucleotide is from RSV, preferably RSV strain B, and the first and the second oligonucleotides comprise, consist or consist essentially of a sequence selected from the list consisting of:
a) SEQ ID NOs: 65 (first oligonucleotide) and 25 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 65 and 25, or
b) SEQ ID NOs: 66 (first oligonucleotide) and 26 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 66 and 26.

In an embodiment, the % of total GC content in the first and/or the second oligonucleotide is about 20, 30, 40, 45, 50, 55, 60, 65, or 70%. In a preferred embodiment, the % of total GC content present in the first and/or the second oligonucleotides ranges from 20 to 80%, preferably between 30% to 80%, 40% to 80%, 45% to 80%. In an embodiment, the % of total GC content in the first and/or the second oligonucleotide is of at least 35%, 40%, 45%, 50%, or 55%. Most preferably, the % of total GC content in the first and/or the second oligonucleotide is between 40 to 80%.

The first and/or the second oligonucleotides may be natural or chemically modified (i.e., non-naturally occurring). The sugar groups of the nucleotide subunits may be ribose, deoxyribose or modified derivatives thereof such as o-methyl ribose. The nucleotide subunits of the first and/or the second oligonucleotides may be joined by phosphodiester linkages, phosphorothioate linkages, methyl phosphonate linkages or by other rare or non-naturally occurring linkages that do not prevent hybridization of the second oligonucleotide. In an embodiment, the polypyrimidine region of the parallel clamp further comprises any of the nucleoside derivatives that have been described to stabilize the triplex such as, but not limited to, 5-methyl-C, 5-bromo-U, 5-propynyl-C, 5-propynyl-U, pseudo-U, pseudoiso-C, pyrido[2,3-d]pyrimidine, benzo[f]quinazoline, 6-oxo-C, 6-amino-C, 2-aminopyridine, 8-oxo-A, N7-alkyl-G.

Furthermore, the first and/or the second oligonucleotides may have uncommon nucleotides or non-nucleotide moieties. The first and/or the second oligonucleotide as defined herein is a nucleic acid, preferably DNA, but may be RNA or have a combination of ribo- and deoxyribonucleotides covalently linked.

Both steps i) and ii) comprise contacting the oligonucleotides as defined in any of the embodiments above with a test sample. In an embodiment, the sample is an isolated biological sample. In a preferred embodiment, said isolated biological sample may include, but is not limited saliva, tears, urine, blood, serum, plasma, sweat, vaginal fluids, semen, feces, mucous, breast milk, ascites, lymph, pleural effusion, synovial fluid, bone marrow, cerebro-spinal fluid, and washings from bodily cavities (e.g., bronchial lavage), nasopharyngeal swabs, hair, tissue, bones, or teeth. In a preferred embodiment, the isolated biological sample is selected from the group consisting of tears, saliva, or mucous, mucous, nasopharyngeal swabs or washings from bodily cavities (e.g., bronchial lavage).

In an embodiment, the steps of contacting the first and the second oligonucleotides with the test sample can be performed in different manners. In an embodiment, the contact of the first and the second oligonucleotide with test sample can be simultaneously or sequentially. In another embodiment, the is contacted with the first oligonucleotide first, and then the first oligonucleotide alone or forming a complex with the target polynucleotide is contacted with the second oligonucleotide. In another embodiment, the test sample is contacted with the second oligonucleotide first, and then the second oligonucleotide alone or forming a complex with the target polynucleotide is contacted with the first oligonucleotide. It is noted that the complex formed between the first or the second oligonucleotides, or both, with the target polynucleotide, can occur before applying the test sample to the system or device where the method is being implemented, or it can occur once the test sample has been added to said system or device.

The oligonucleotides of step i) and ii), may comprise one or more chemical modifications at their 3' and/or 5' end to allow the detection step or, for example, to make them suitable for use in a device or in a system. Thus, in an embodiment, the first or the second oligonucleotides of steps i) and ii), or both, may be modified to comprise other molecules, such as capture tag or reporter molecules. This is developed in detail below in step iii).

Step iii) of the first aspect comprises detecting the hybridization between the first and the second oligonucleotides to the at least one target polynucleotide. As explained above, the *in vitro* method of the present invention allows the detection of at least one target polynucleotide. Said detection is performed only when both the first and the second oligonucleotides have successfully hybridized with the at least one target polynucleotide that wants to be detected. In an embodiment, said detection of step iii) is performed by means of a reporter molecule. A "reporter molecule" or "reporter probe" is referred herein as a molecule that allows the chemical, physical, biological, thermal, visual, or any other type of detection of the binding of the first oligonucleotide and the second oligonucleotide to the at least one target polynucleotide. By visual detection is herein understood as any detection that can be distinguished by the naked eye without needing to use any type of instrumentation for detection such as an infrared camera.

In an embodiment, the reporter molecule is conjugated to the first or the second oligonucleotide, or both. By "conjugated " is referred to the association of two chemical moieties or molecules. Preferably, the reporter molecule is conjugated to the first oligonucleotide. In an embodiment, the reporter molecule is conjugated to the first or to the second region of the first oligonucleotide. In an embodiment, the reporter molecule is conjugated to the 3' and/or the 5' ends of the first or the second oligonucleotide, or to both. The reporter molecule may be conjugated by, for instance, covalent bonds, electrostatic interactions or by using intercalating agents. Preferably, the reporter molecule is conjugated by covalent bonds.

To conjugate the reporter molecule to the first or the second oligonucleotide, it may be necessary that the first or the second oligonucleotide are chemically modified to allow the conjugation. Said modifications include, but are not limited to, amine groups, thiol groups, carboxylic acid groups, maleimido, alkyne, alkene, diene, azido, tetrazine groups, etc.

In an embodiment, the reporter molecule allows the detection of the binding of the first and the second oligonucleotides to the at least one target polynucleotide by means of, included but not limited to, radioactive, colorimetric, fluorescence, thermal dissipation, or electrochemical methods. In an embodiment, the reporter molecule is selected from the group of bioconjugates, fluorophores, fluorochromes, chromophore, dyes, chemiluminiscent reagents, enzymes, enzymatic substrates, cofactors, enzymatic inhibitors, particles, radioactive isotopes, proteins, or antibodies. Preferably, the reporter molecule is selected from the group of fluorophores, enzymes, biotin and nanoparticles.

In a preferred embodiment, said reporter molecule comprises or consists of nanoparticles. In an embodiment, the nanoparticles are capable of emitting heat by surface plasmon resonance upon stimulation by a light source, thereby allowing the detection step iii) to be carried out by means of thermal dissipation detection. The nanoparticles as reporter molecules can be conjugated to the first or to the second oligonucleotides. In the context of the present invention, metal nanoparticle having a surface plasmon band is understood as any mono- or polycrystalline cluster of metal atoms in any of their oxidation states, or any of their alloys, having all geometric dimensions between 1 and 1000 nm, preferably between 1 and 200 nm. In a preferred embodiment of the invention, said metal atoms are noble metals. In a more preferred embodiment of the invention, said metal atoms are gold, silver or copper atoms. In a preferred embodiment, the nanoparticles are magnetic nanoparticles. In an even more preferred embodiment of the invention, the nanoparticles are tubular, spherical, square or triangular in shape. In a preferred embodiment, the nanoparticles are gold nanoprisms. In an embodiment, the reporter molecule comprises or consists of nanoparticles, preferably gold nanoprisms, that are conjugated to the first oligonucleotide. Examples of suitable nanoparticles can be found in ES201231209 and protocols for the gold surface functionalization with oligonucleotides can be found in Anna Aviñó et al. 2019. doi: 10.1002/cpnc.78 (basic protocol 6). It is noted that nanoparticles as referred herein can also be used as capture tags, as will be further developed below. In an embodiment, the first or the second oligonucleotides are functionalized with amino groups to be conjugated to the nanoparticles. In an embodiment, the nanoparticles are functionalized with carboxyl groups.

In another embodiment, the reporter molecule is biotin and the detection is performed by adding streptavidin or avidin covalently conjugated to an enzyme, such as horseradish peroxidase, and then measuring the signal once the appropriate enzymatic substrate is added. The biotin as reporter molecule can be conjugated to the first or to the second oligonucleotides. In another embodiment, the reporter molecule comprises or consists of an enzyme. Preferably, said enzyme is an enzyme capable of producing an electroactive product when an appropriate enzyme substrate is applied thereby allowing the detection of step iii) to be carried out by electrochemical detection. An enzyme as reporter molecules can be conjugated to the first or to the second oligonucleotides. In another embodiment, the reporter molecule is conjugated to the second oligonucleotide and it is horseradish peroxidase and the detection is performed by adding the organic substrate that allows having the appropriate enzymatic signal without the need of using biotin-streptavidin. In another embodiment, the reporter molecule is conjugated to the second oligonucleotide and it is alkaline phosphatase and the detection is performed by adding the appropriate enzymatic substrate.

In another embodiment, the reporter molecule comprises or consists of one or more chromophore or fluorophores. In an embodiment, when the reporter molecule comprises or consists of one or more chromophore or fluorophore, the detection of step iii) may be carried out by exciting the fluorophores with a laser beam of a defined wavelength. A chromophore or fluorophore as reporter molecule can be conjugated to the first or to the second oligonucleotides. In an embodiment, the reporter molecule is conjugated to the second oligonucleotide and said reporter molecule is a chromophore or a fluorophore such as TAMRA, Cy3, Cy5, fluorescein or others, and the detection is performed upon energy excitation and emission by, for instance, measuring the signal in a fluorescence scanner. In an embodiment, the reporter molecule is a reporter probe, i.e., another oligonucleotide that is conjugated to a detection molecule and that hybridizes with the first or the second oligonucleotides.

In an embodiment, the first and/or the second oligonucleotide are further modified to be conjugated to a capture tag. In the context of the present invention "capture tag" (e.g. biotin, nanoparticles, amino groups, carboxyl groups) is understood as the modification at the 3' and/or 5' ends of the first or the second polynucleotide that allows binding with a capture molecule or to the surface of solid support. In a preferred embodiment, the capture tag is selected from the group consisting of biotin, nanoparticles, amino groups or carboxyl groups. In an embodiment, the capture tag can be a capture probe, i.e., an oligonucleotide that hybridizes with the first or the second oligonucleotides. In the context of the present invention "capture molecule" is understood as recognition molecule (e.g. streptavidin, avidin, neutravidin) immobilized to a substrate or solid support, for instance to the detection line of the lateral flow system or device, with the capability to bind one of the ends of the first or the second oligonucleotide that was previously modified with a capture tag. This binding is carried out by a biorecognition process. In an embodiment, the capture molecule comprises or consists of streptavidin, avidin, or neutravidin.

In an embodiment, the capture tag is conjugated to the first or the second oligonucleotide, or both. In another embodiment, the capture tag is conjugated to the 3' and/or the 5' ends of the first or the second oligonucleotides. In an embodiment, the capture tag is conjugated to the first or to the second region of the first oligonucleotide. In a preferred embodiment, the capture tag is biotin. In a preferred embodiment, the capture tag is a nanoparticle.

In order to conjugate the capture tag to the first or the second oligonucleotide, it may be necessary that the first or the second oligonucleotide are chemically modified to allow the linkage or conjugation. Said modifications include, but are not limited to, amine groups, thiol groups, carboxylic acid groups, maleimido, alkyne, alkene, diene, azido, tetrazine groups, etc.

Further, it could also be that the linkage or conjugation of the first or the second oligonucleotides to the solid support or substrate is performed by means of chemical modification of the oligonucleotides, without the need of further adding more molecules. In this case, said chemical modifications are considered capture tags, since they allow binding with to the surface of a solid support in order to immobilize the oligonucleotides. Thus, in an embodiment, the first and the second oligonucleotide are modified to allow the conjugation or linkage, and said modifications include, but are not limited to, amine groups, thiol groups, carboxylic acid groups, maleimido, alkyne, alkene, diene, azido, tetrazine groups, etc. In a preferred embodiment, the first or the second oligonucleotides are modified to include an amino group to be able to be immobilized to the glass surface of the system or device or to magnetic nanoparticles.

In a preferred embodiment, the first oligonucleotide is conjugated to a reporter molecule, and the second oligonucleotide is conjugated to a capture tag, or the other way around. Fig. 2 depicts the presence of reporter molecules and capture probes or tags conjugated to the oligonucleotides according to the method provided herein. It should be noted that a specific molecule or compound can be used both as capture tag or as reporter molecule, depending of the chosen strategy for the design of the first and second oligonucleotides according to the present invention. For example, in a preferred embodiment, the first oligonucleotide is conjugated to a nanoparticle, and the second oligonucleotide is biotinylated. In this preferred case, the nanoparticle can be either the reporter molecule by means of surface plasmon resonance and the biotin can be the capture tag by binding to the streptavidin, avidin or neutravidin (capture molecule) immobilized in the test trip, or it can be the other way around: in an embodiment, the nanoparticle conjugated to the first oligonucleotide may be the capture tag and the biotin conjugated to the second oligonucleotide may act as a reporter molecule upon addition of streptavidin, avidin or neutravidin.

It is noted that the different embodiments described in steps i), ii) and iii) can be combined with each other. Thus, any possible combination of the first oligonucleotide as defined in step i), with the second oligonucleotide as defined in step ii), and with the detection method, reporter molecules, capture tags and capture molecules as defined in step iii) is understood to be included in the present invention.

In the context of the present invention, the *in vitro* method of the first aspect can optionally comprise a further step, herein after referred to as step iv), in which the amount of target polynucleotide detected in step i) to iii) is quantified. Thus, in an embodiment, the *in vitro* method according to the first aspect or any of its embodiment comprises the steps of:
i) contacting the test sample with a first oligonucleotide, wherein the first oligonucleotide comprises a first polypurine region and a second region, wherein said first and second regions are connected by a linker molecule, and wherein:
   a) the second region is a polypurine region and said first and second regions are capable of forming a hairpin maintained by reverse-Hoogsteen bonds with the two strands running in antiparallel orientation, or
   b) the second region is a polypyrimidine region and said first and second regions are capable of forming a hairpin maintained by Hoogsteen bonds with the two strands running in parallel orientation;

   wherein the first region is of at least 12 nucleotides in length and wherein at least 6 nucleotides of the at least 12 nucleotides of the first region are engaged forming said hairpin with the second region; and
   wherein the first region is substantially complementary to a polypyrimidine target region comprised in the at least one target polynucleotide, wherein the hybridization between the first polypurine region and the polypyrimidine target region forms a structure comprising a triplex; and
ii) contacting the test sample with a second oligonucleotide, wherein the second oligonucleotide is substantially complementary to the at least one target polynucleotide,
iii) detecting the hybridization between the first and the second oligonucleotides to the at least one target polynucleotide,
iv) quantifying the amount of target polynucleotide present in the test sample.

It should be noted that the embodiments defined above for steps i), ii), and iii), can be combined with the embodiments of step iv), which are explained below:
Step iv) of the first aspect is an optional step and comprises quantifying the amount of target polynucleotide present in the test sample, which is preferably an isolated biological sample. In the quantification step, the total or relative amount of target polynucleotide can be inferred. The quantification step iv) of the method will vary depending on the reporter molecule used in step iii). A skilled person in the art would be able to choose the adequate quantification technique depending on the reporter molecule used. For instance, if nanoparticles conjugated to the first or the second oligonucleotide are used, the analytical result can be read and measured from the color change due to nanoparticle's aggregation and/or the measurement of the emitting heat by surface plasmon resonance upon stimulation by a light source. If a biotinylated first or second oligonucleotide is used, the quantification can be performed by adding streptavidin or avidin covalently conjugated to an enzyme, such as horseradish peroxidase, and then measuring the signal once the appropriate enzymatic substrate is added. If the first or the second oligonucleotide is linked to an enzyme such as horseradish peroxidase or alkaline phosphatase, quantification can be carried out by measuring the signal once the appropriate enzyme substrate is added. If a fluorophore is used to label the first or the second oligonucleotide the quantification may directly performed by measuring the signal in a fluorimeter (in the case of the microarray format the slide is read using a fluorescence scanner).

In an embodiment, a reference value may be or not needed in order to quantify the amount of target polynucleotides present in the test sample. The "reference value" may derive from a test sample collection or an individual sample where the concentration of the at least one target polynucleotide is known. In an embodiment, the reference value consists of a value obtained in a sample spiked with known concentrations of the at least one target polynucleotide that wants to be detected in the test sample. If the comparison with a reference value is needed, the value obtained after the detection step is compared with the reference value, detecting an increase or a decrease in the amount of target polynucleotide.

Several known systems or devices may be used to implement the method of the present invention. They may include, but are not limited to, point-of-care (POC) tests for the rapid detection of the at least one target polynucleotide. The POC tests are usually designed as easy-to-use membrane-based test strips, often enclosed by a plastic test cassette. An example of a POC test is a test based on immunochromatographic assays, particularly Lateral flow tests (LFTs), which operate on the same principles as the enzyme-linked immunosorbent assays (ELISA). In essence, these the LFTs run the test sample along the surface of a pad with reactive molecules that show a positive or negative result. The result may be displayed visually or upon stimulation with, for instance, a laser. Lateral flow-based biosensors usually comprise at least three parts: sample pad, nitrocellulose membrane, and absorbent pad. Further, the method disclosed herein can also be implemented in other devices or systems, such as DNA microchip arrays or electrochemical biosensors.

In view of this, in a second aspect, the present invention provides a system or a device suitable for implementing the method according to the first aspect or any of its embodiments. In the context of the present invention, a "system" is understood as a group of interacting or interrelated elements where the method is implemented. The system can comprise at least a device, together with other elements or reagents (such as first oligonucleotide, second oligonucleotide, etc) necessary for implementing the method. In an embodiment, the system or the device suitable for implementing the method for detecting the at least one target polynucleotide in a test sample, preferably an isolated biological sample, comprises:
i) a first oligonucleotide, wherein the first oligonucleotide comprises a first polypurine region and a second region, wherein said first and second regions are connected by a single-stranded nucleotide region, and wherein:
   a) the second region is a polypurine region and said first and second regions are capable of forming a hairpin maintained by reverse-Hoogsteen bonds with the two strands running in antiparallel orientation, or
   b) the second region is a polypyrimidine region and said first and second regions are capable of forming a hairpin maintained by Hoogsteen bonds with the two strands running in parallel orientation;

   wherein the first region is of at least 12 nucleotides in length and wherein at least 6 nucleotides of the at least 12 nucleotides of the first region are engaged forming said hairpin with the second region; and
   wherein the first region is substantially complementary to a polypyrimidine target region comprised in the at least one target polynucleotide, wherein the hybridization between the first polypurine region and the polypyrimidine target region forms a structure comprising a triplex; and
ii) a second oligonucleotide, wherein the second oligonucleotide is substantially complementary to the at least one target polynucleotide; and
iii) optionally, a reporter molecule, as defined in the first aspect or any of its embodiments.

That is, the second aspect relates to a system or device suitable for implementing the method according to the first aspect or any of its embodiments, wherein said device or system is characterized by comprising the first oligonucleotide, the second oligonucleotide, and, optionally, a reported molecule and/or capture tag, as have been defined in the first aspect or any of its embodiments.

In an embodiment, the first oligonucleotide comprised in the system or device of the second aspect comprises a first polypurine region and a second region form a hairpin and are connected by a linker molecule, preferably by a single-stranded region, most preferably by a thymidine-rich region, as explained above in the first aspect. In an embodiment, the first polypurine region of the first oligonucleotide comprised in the system or device of the second aspect is at least 12, 13, 14, 15, 16, 17, 18, 19, 20, 21, 22, 23, 24, 25, 30, or 35 nucleotides in length. In another embodiment, said first polypurine region has a length ranging from 12-20, 12-25, 15-25, 20-25, 20-30, 20-35 nucleotides or any value in between. In an embodiment, the second region of the first oligonucleotide comprised in the system or device of the second aspect is at least 6, 7, 8, 9, 10, 11,12, 13, 14, 15, 16, 17, 18, 19, 20, 21, 22, 23, 24, 25, 30, or 35 nucleotides in length. In another embodiment, the second region has a length ranging from 6-20, 12-20, 12-25, 15-25, 20-25, 20-30, 20-35 nucleotides or any value in between. In a preferred embodiment, the second region may be a polypurine region (PPRH) or a polypyrimidine region (parallel clamps). Also, preferably, the second region is a polypurine region as it consists of a succession of consecutive purines.

In a preferred embodiment, the total length of the first oligonucleotide comprised in the system or device of the second aspect is at least 21, 30, 35, 40, 45, 50, 60, 70, 80, 90, or 100 nucleotides. In a preferred embodiment, the total length of the first oligonucleotide is between 21 to 100, 30 to 100, 40 to 100, or 50 to 100 nucleotides. As stated above, the first polypurine region and the second region are capable of forming a hairpin. If the first oligonucleotide is a PPRH, the second region is a polypurine region and therefore the first polypurine region and the second polypurine region form an antiparallel hairpin wherein both regions run in an antiparallel orientation. If the first oligonucleotide is a parallel clamp, the second region is a polypyrimidine region and therefore the first polypurine region and the second polypyrimidine region form a parallel hairpin wherein both regions run in a parallel orientation. Preferably, the first oligonucleotide comprised in the system or device according to the second aspect is a PPRH, wherein, preferably, all the purines in the first polypurine region and all the purines in the second region are bound with reverse-Hoogsteen bonds.

In an embodiment, the first oligonucleotide comprised in the system or device according to the second aspect comprises, consists, or consists essentially of a sequence selected from the group consisting of SEQ ID NOs: 60, 62, 63, 64, 67, 68, 65, or 66, or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 60, 62, 63, 64, 67, 68, 65, or 66.

The second oligonucleotide comprised in the system or device of the second aspect is substantially complementary, preferably 95%, more preferably 100% complementary, to the target polynucleotide. Said second oligonucleotide comprises at least 12, 20, 25, 30, or 35 nucleotides in length and is substantially complementary, preferably about 85%, 90%, 95%, 96%, 97%, 98%, 99%, or 100% complementary to the target polynucleotide. In an embodiment, the second oligonucleotide comprised in the system or device of the second aspect comprises, consists, or consists essentially of a sequence selected from the group consisting of SEQ ID NOs: 5, 12, 13, 19, 25, 26, 33, or 34 or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 5, 12, 13, 19, 25, 26, 33 or 34.

In a preferred embodiment, the first polypurine region of the first oligonucleotide comprised in the system or device of the second aspect is at least 70%, 80%, 90%, 92%, 95%, 96%, 97%, 98%, 99% or, preferably, 100% complementary, over its full length, to the polypyrimidine target region comprised in the at least one target polynucleotide. In an embodiment, the polypyrimidine target region comprises a succession of pyrimidines with none, one, two, three or more purine interruptions. In an embodiment, a maximum of three purine interruptions in the polypyrimidine target region is allowed. In an embodiment, the polypyrimidine target region has between 12 nucleotides and the maximum possible length with the first polypurine region, preferably at least 20 nucleotides, more preferably at least 24 nucleotides, most preferably at least 30 nucleotides. The first and/or the second oligonucleotides comprised in the system or device or system may be natural or chemically modified (i.e., non-naturally occurring).

In an embodiment according to the second aspect or any of its embodiments, the at least one target polynucleotide is from a coronavirus, preferably a SARS coronavirus, more preferably SARS-CoV-2 virus, and the first and the second oligonucleotides comprise, respectively, a sequence selected from the list consisting of:
a) SEQ ID NOs: 62 (first oligonucleotide) and 5 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 62 and 5,
b) SEQ ID NOs: 60 (first oligonucleotide) and 5 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 60 and 5,
c) SEQ ID NOs: 63 (first oligonucleotide) and 12 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 63 and 12,
d) SEQ ID NOs: 63 (first oligonucleotide) and 13 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 63 and 13, or
e) SEQ ID NOs: 64 (first oligonucleotide) and 19 (second oligonucleotide), or a sequence with at least 50%, 60%, 70%, 80%, 85%, 90%, 91%, 92%, 93%, 94%, 95%, 96%, 97%, 98%, 99% or 100% sequence identity over the full length with SEQ ID NOs: 64 and 19.

In an embodiment according to the second aspect or any of its embodiments, the at least one target polynucleotide is from Influenza virus, preferably Influenza H1N1 virus, and the first and the second oligonucleotides comprise, respectively, a sequence selected from the list consisting of:
a) SEQ ID NOs: 67 (first oligonucleotide) and 33 (second oligonucleotide), or a sequence with at least 90% sequence identity over the full length with SEQ ID NOs: 67 and 33, or
b) SEQ ID NOs: 68 (first oligonucleotide) and 34 (second oligonucleotide), or a sequence with at least 90% sequence identity over the full length with SEQ ID NOs: 68 and 34.

In an embodiment according to the second aspect or any of its embodiments, the at least one target polynucleotide is from RSV, preferably RSV strain B, and the first and the second oligonucleotides comprise, respectively, a sequence selected from the list consisting of:
a) SEQ ID NOs: 65 (first oligonucleotide) and 25 (second oligonucleotide), or a sequence with at least 90% sequence identity over the full length with SEQ ID NOs: 65 and 25, or
b) SEQ ID NOs: 66 (first oligonucleotide) and 26 (second oligonucleotide), or a sequence with at least 90% sequence identity over the full length with SEQ ID NOs: 66 and 26.

In an embodiment, the device or system comprises a surface or support to immobilize the first or the second oligonucleotides. The surface or support may be a solid support such as membrane, plastic, surface of nanoparticles, or glass in order to implement the method of the first aspect or any of its embodiments into a system or device. To do so, it may be necessary to further modify either the first or the second oligonucleotides, or even both, to include at least a capture tag conjugated to any of them that allows their immobilization on the surface of the test device or on a solid surface, as defined above. Thus, in an embodiment, the system further comprises iv) at least a capture tag and/or a capture molecule.

In an embodiment according the second aspect or any of their embodiments, the system or device is a lateral flow system or device, preferably used to implement the method of the first aspect. In a preferred embodiment, the detection step iii) of the method as defined in the first aspect above that is implemented in said lateral flow system or device is performed by means of thermo lateral flow assay methods, preferably by thermo lateral flow assay methods, wherein the system or device further comprises a thermal sensor with a capture molecule and a heat sensitive surface (Fig. 8a). In an embodiment, the capture molecule of the sensor may be immobilized on a support with a heat sensitive surface by physical retention inside a matrix (entrapment), physical adsorption on a matrix by means of ionic or hydrophobic interactions or binding by means of covalent bond.

If the device or system comprises a support with a heat sensitive surface is understood as any surface capable of experiencing a structural change when heated, resulting in image development. Thermal paper or ink which will give rise to a development signal after being subjected to a temperature increase due to its heat sensitivity will preferably be used as the heat sensitive surface. Thermal paper is considered as any paper having a thermal layer incorporating a dye, a sensitizer and a color developer (regardless of the chemical components with which it has been prepared) capable of reacting with one another giving rise to an image after being subjected to a temperature increase. In another preferred embodiment, any smart polymer which, after being subjected to an external stimulus such as temperature change, gives rise to a response in the polymer properties such as: contraction, bending, color change, state change, luminescence, etc., will be used as the heat sensitive surface. The types of temperature sensitive polymers include: PNIPAM, poly(N-isopropylacrylamide), poly(N-vinylpiperidine) or poly(N-vinylcaprolactam).

In one embodiment of the invention, the support with a heat sensitive surface of the system comprises at least one heat sensitive support where molecular recognition takes place, such as a membrane made of cellulose or derivatives thereof (cellulose nitrate, cellulose acetate, etc.) or heat sensitive paper.

In another embodiment, the support with a heat sensitive surface of the system comprises two supports. A first support where the first or the second oligonucleotides as defined in the first aspect or any of its embodiments will be immobilized, and said support is a membrane made of cellulose or derivatives thereof (cellulose nitrate, cellulose acetate, etc.), or other materials such as polystyrene, cyclo olefin polymer, polydimethysiloxane, ceramic, silicon or glass. A second support consisting of a heat sensitive surface which will give rise to the development signal after being subjected to a temperature increase will be adhered to this first support.

Next, the sample fluid is transported to the membrane and the capture molecule, preferably streptavidin, immobilized in the test line/s will recognize the capture tags located at one of the end of the first or second oligonucleotide, preferably the second oligonucleotide. When the capture molecule and the capture tag are bound, the at least one target polynucleotide to be detected and comprised in the test sample will be retained in the test line of the device or system. The determination of the presence or absence of said target polynucleotide is determined within the same lateral flow system or device similarly using the reporter molecule that is also located at one of the end of the first or the second oligonucleotide, preferably the first oligonucleotide. The system will provide a readout signal that can be evaluated by naked eye or by a specialized instrument, such an infrared camera (see Fig. 8). In another embodiment, a visual detection method is used with the LFT, in which the test sample, also referred herein as to sample fluid, is contacted with the first and the second oligonucleotides according to the first aspect of the present invention, and the sample mix is then applied in the sample pad of the LFT.

In another embodiment according the second aspect or any of their embodiments, the system or device is a hybridization microarray or DNA microarray chip system or device, preferably used to implement the method of the first aspect. In a preferred embodiment, the detection step iii) of the method as defined in the first aspect above that is implemented in the DNA microarray chip system or device is carried out by exciting the fluorophores with a laser beam of a defined wavelength. In an embodiment, the DNA microarray chip system or device comprises a flat chip or glass slides on which DNA probes or oligos can be immobilized. In an embodiment, the first or the second oligonucleotides as described in the first aspect or any of its embodiments and comprised in the system or device of the second aspect are immobilized to the chip or slide. Since the at least one target polynucleotide will naturally bind with its complementary first or second oligonucleotide comprised in the system or device, in an embodiment, the chip may be used to identify the presence of particular target polynucleotide in a test sample. These chips, containing hundreds or thousands of unique oligonucleotides, are also called DNA microarrays and can be manufactured using a variety of techniques, including semiconductor processing technology, on a variety of surfaces, including glass and plastic.

DNA microarray technology is a platform for the simultaneous analysis of complex fluids, in a high throughput manner, allowing the analysis of many test samples at the same time (high throughput capabilities) and the simultaneous detection of several targets (multiplexation). Each spot in the array represents an individual and independent assay for the detection of a given analyte. Furthermore, since a standard curve is performed in each assay, this format enables the quantification of the target that is being detected. Thus, in an embodiment according to the second aspect or any of its embodiments, the system or device comprises or consists of a microarray chip system or device. In an embodiment according to the second aspect or any of its embodiments, the microarray chip system or devices comprises immobilized biomolecules onto flat substrates, which can be of different chemical nature including SiO₂, Si₃N₄, gold, etc. A collection of small volume spots attached to a solid surface will act as recognition elements for the target that in a specific manner will be detected. The principle behind this methodology is based on sequence complementarity. DNA molecules printed onto the slide surface act as probes that hybridize selectively with the target. A quantifiable signal is raised by labeling the first or the second oligonucleotides, as explained above in the detection step of the method of the first aspect. In a preferred embodiment, the detection step when the method is performed using a DNA microarray chip system or device is carried out by optical methods, preferably by using fluorophores as reporter molecules.

In a preferred embodiment, DNA microarray chip system or device of the second aspect provides a readout signal that can be scanned with a microarray scanner to visualize and quantify fluorescence after excitation with a laser beam of a defined wavelength. Other readout protocols could be used if the nature of the reporter molecule varies. Therefore, in an embodiment, devices according to the second aspect or any of its embodiments allow high throughput detection of different target polynucleotides when the method of the first aspect or any of its embodiments is implemented into the system or device. In an embodiment, the DNA microarray chip according to the second aspect or any of its embodiments is used to detect at the same time, e.g., simultaneously, at least one, preferably two or more, target polynucleotides comprised in the test sample, preferably the isolated biological sample.

In a preferred embodiment, the solid surface comprised in the system or device is a glass microscope slide functionalized with a capture molecule, and this flat substrate can further comprise nitrocellulose membranes and other synthetic papers. The immobilization strategies underlie a surface chemistry, that in this case is based on the derivatization of the hydroxyl groups of the glass slide with a silane reagent that contains a terminal isocyanate group by which the amino group of the oligonucleotide is linked. Other oligonucleotide modifications are also possible (such as thiol groups) when working with other immobilization approaches. In a preferred embodiment, the first oligonucleotide and/or the second oligonucleotide comprised in the system or device is immobilized to the glass surface through modifying it by adding an amino group at their 3' and/or 5' end.

In another embodiment according the second aspect or any of their embodiments, the system or device is an electrochemical system or device, preferably used to implement the method of the first aspect. In a preferred embodiment, the detection step iii) of the method as defined in the first aspect above that is implemented in the electrochemical system or device is carried out by electrochemical detection. In the case of an electrochemical system or device, it is characterized by comprising a compact fluidic biosensor system that comprises a reusable array comprising at least two individual electrochemical cells that are individually addressable, a disposable fluidic component, preferably made of paper (cellulose or nitrocellulose), for analysis under capillary flow conditions, and a cartridge to integrate and align the array and the fluidic component. In an embodiment, the at least two individual electrochemical cells are aligned and in contact with individual paper fluidic channels of the disposable fluidic component, so that electrochemical cells can detect electrochemical reactions taking place in each paper fluidic channel. In a preferred embodiment, the compact fluidic device integrates a chronoamperometric detection approach.

The system or device further comprises one or more working electrodes and at least one counter/reference electrode common to all the electrochemical cells, such that each electrochemical cell includes one working electrode and a defined area of the shared counter/reference electrode. The counter/reference electrode is used as counter electrode and at the same time as reference electrode for the electrochemical analysis.

In an embodiment, the one or more working electrodes are aligned in a longitudinal straight direction, and the shared counter/reference electrode is a straight track, such that the working electrodes are adjacent to one side of the shared counter/reference electrode. In addition, the longitudinal straight direction of the working electrodes alignment, is parallel to the counter/reference electrode (two-electrode configuration).

Preferably, the fluidic channels are also aligned in a straight longitudinal direction, following the parallel arrangement of the counter/reference electrode of the first embodiment.

In an embodiment, the system or device further comprises a set of connection pads, and a set of connection tracks connecting the working electrodes, shared counter/reference electrode, with the connection pads. Preferably, a part of each connection track is parallel to the counter/reference electrode, and to the shared counter and shared reference electrodes.

In an embodiment, the system or device is a compact fluidic device, wherein liquids flow by capillary action on small channels and different sensor approaches could be implemented for making them more rapid and user-friendly. The main application of these devices is on-site, in field analysis at the point-of-care, point-of-need or at the counter, avoiding the need to collect the test sample, stabilize it and transport it to a centralized laboratory for further analysis.

As explained above, the systems or devices according to the second aspect or any of its embodiments may be used for implementing the method of the first aspect. Therefore, any embodiment of the first aspect, particularly the embodiments referred to the first and the second oligonucleotides, should be considered an embodiment comprised in the system or device of the second aspect. Particular preferred embodiments of the system or device according to the second aspect where the method of the first aspect is implemented are described below:

In a particular embodiment, when the method according to the first aspect or any of its embodiments is implemented using a lateral flow system or device (LFA), preferably a thermo lateral flow system or device (thermo LFA), such LFA is characterized by comprising a first oligonucleotide conjugated to at least a reporter molecule and a second oligonucleotide conjugated to at least a capture tag. Said reporter molecule can be nanoparticles that are capable of emitting heat by surface plasmon resonance upon stimulation by a light source, thereby allowing the detection step iii) to be carried out by means of thermal dissipation detection. Preferably, said nanoparticles conjugated to the first oligonucleotide are gold nanoprisms. Preferably, the first oligonucleotide is functionalized with amine groups. Preferably, the capture tag is biotin. Also, preferably, the lateral flow comprises a line comprising at least a capture molecule, such as, but not limited to streptavidin, that is used to bind the capture tag conjugated to the second oligonucleotide so that when the capture tag (e.g. biotin) binds to the capture molecule (e.g. streptavidin) the second oligonucleotide alone, or forming a complex with the first oligonucleotide and target polynucleotide, is anchored or retained in the pad of the thermo LFA. In a preferred embodiment, the test sample is contacted and pre-incubated with the first and the second oligonucleotides (which are preferably conjugated to the reporter molecule (e.g. nanoparticles) and to the capture tag (e.g. biotin), respectively), before applying it to the strip of the thermo LFA device. The system or device described herein is exemplified in example 7 and it is referred to as "System 1".

In a particular embodiment, when the method according to the first aspect or any of its embodiments is implemented using a lateral flow system or device (LFA), preferably a thermo lateral flow system or device (thermo LFA), such LFA is characterized by comprising second oligonucleotide conjugated to at least a reporter molecule and a first oligonucleotide conjugated to at least a capture tag. Said reporter molecule can be nanoparticles, such as gold nanoprisms, that are capable of emitting heat by surface plasmon resonance upon stimulation by a light source, thereby allowing the detection step iii) to be carried out by means of thermal dissipation detection. Preferably, the capture tag is biotin. Preferably, the lateral flow further comprises a line comprising at least a capture molecule, such as, but not limited to streptavidin, that is used to bind the capture tag conjugated to the first oligonucleotide so that when the capture tag (e.g. biotin) binds to the capture molecule (e.g. streptavidin), the first oligonucleotide alone, or forming a complex with the second oligonucleotide and target polynucleotide, is anchored to the thermo LFA. Preferably, the second oligonucleotide is functionalized with amine groups. Preferably, the first oligonucleotide is functionalized with carboxy groups. In a preferred embodiment, the test sample is contacted and pre-incubated with the second oligonucleotide (which is preferably conjugated to the reporter molecule (e.g. nanoparticles). Then, the mixture (test sample and second oligonucleotide) is applied to the strip of the thermo LFA device and will then be contacted with the first oligonucleotide, since it was previously immobilized in the test line of the strip of the thermo LFA system or device. The system or device described herein is exemplified in example 7 and it is referred to as "System 2". Examples of a LFT using nanoparticles in thermal biosensors are described in ES201231209.

In a particular embodiment, when the method according to the first aspect or any of its embodiments is implemented using a DNA microarray chip system or device, such DNA microarray chip system or device is characterized by comprising a first oligonucleotide conjugated to at least a capture tag that immobilizes it to the surface of the microarray device, and a second oligonucleotide conjugated to at least a reporter molecule. The capture tag is preferably an amino group functionalized to the first oligonucleotide. The reporter molecule preferably comprises or consists of one or more fluorophores, thereby allowing the detection step iii) to be carried out by exciting the fluorophores with a laser beam of a defined wavelength. In a preferred embodiment the reporter molecule conjugated to the second oligonucleotide comprised in the device or system is selected from the group including, but not limited to, fluorophores (e.g. TAMRA, Cy3), chromophore, enzymes or chemiluminescent reagents or enzymatic substrates.

In a particular embodiment, when the method according to the first aspect or any of its embodiments is performed using the electrochemical system or device, such electrochemical system or device is characterized by comprising a second oligonucleotide conjugated to at least a reporter molecule and a first oligonucleotide conjugated to at least a capture tag. The reporter molecule may be an enzyme, preferably an enzyme such as horseradish peroxidase or alkaline phosphatase, that is used in combination with an appropriate enzyme substrate to produce an electroactive product, thereby allowing the detection step iii) to be carried out by electrochemical detection. Thus, the enzyme produces an electroactive product that is measured with the integrated electrochemical device. The capture tag, preferable an amino group, covalently attaches the first oligonucleotide to magnetic nanoparticles, preferably carboxylated magnetic nanoparticles. In another embodiment, the test sample is mixed with the magnetic nanoparticles conjugated with the first oligonucleotide and with the second oligonucleotide conjugated to an enzyme before adding the mixture to the fluidic component of the device.

Both the first and the second oligonucleotides must bind specifically to the polypyrimidine target region comprised in the test sample. It is noted that, in any of the embodiments of the second aspect, it should be understood that the steps of contacting of the first or the second oligonucleotides with the test sample according to the first aspect or any of its embodiments can occur prior applying the test sample to the system or device, at the same time as the test sample is contacted with the system or device, or after the test sample has been contacted with the system or device.

In a **third** aspect, the present invention relates to a kit of parts for detecting a target polynucleotide in a test sample, preferably an isolated biological sample, comprising at least two recipients comprising:
i) a first oligonucleotide, wherein the first oligonucleotide comprises a first polypurine region and a second region, wherein said first and second regions are connected by a single-stranded nucleotide region, and wherein:
   a) the second region is a polypurine region and said first and second regions are capable of forming a hairpin maintained by reverse-Hoogsteen bonds with the two strands running in antiparallel orientation, or
   b) the second region is a polypyrimidine region and said first and second regions are capable of forming a hairpin maintained by Hoogsteen bonds with the two strands running in parallel orientation;

   wherein the first region is of at least 12 nucleotides in length and wherein at least 6 nucleotides of the at least 12 nucleotides of the first region are engaged forming said hairpin with the second region; and
   wherein the first region is substantially complementary to a polypyrimidine target region comprised in the at least one target polynucleotide, wherein the hybridization between the first polypurine region and the polypyrimidine target region forms a structure comprising a triplex;
ii) a second oligonucleotide, wherein the second oligonucleotide is substantially complementary to the at least one target polynucleotide;
iii) optionally, a third recipient comprising a reporter molecule as defined in the first or second aspect of the present invention or any of their embodiments, and
iv) optionally, a fourth recipient comprising a at least a capture tag and/or a capture molecule as defined in the first or second aspect of the present invention or any of their embodiments.

In a **fourth aspect,** the present invention relates to the *in vitro* use of the system or the kit as defined in the second and third aspect or any of their embodiments, for detecting a target polynucleotide in a test sample, preferably an isolated biological sample. In an embodiment, the *in vitro* use of the system or the kit in the second and third aspect or any of their embodiments is for implementing the method as defined in the first aspect or any of its embodiments.

### SEQUENCE LISTING

### Oligonucleotides to detect SARS-CoV-2 virus

### ➢ CC1 SYSTEM:

### First oligonucleotides (PPRHs):

**SEQ ID NO 1:** CC1PPRH-amino 
5’NH2-
TTTTTGAGCAGAAGGGTAGTAGAGAGTTTTGAGAGATGATGGGAAGACGA
G-3’
**SEQ ID NO 2:** CC1PPRH (gel assays) 5'-GAGCAGAAGGGTAGTAGAGAGTTTTGAGAGATGATGGGAAGACGAG-3'
**SEQ ID NO 60:** CC1PPRH 
5'-NH2- GAGCAGAAGGGTAGTAGAGAGNGAGAGATGATGGGAAGACGAG -
3'
 Wherein "N" is a linker molecule, preferably a single-strand nucleotide, preferably a thymidine-rich, most preferably between 3 to 5 thymidines.
**SEQ ID NO 3:** CC1duplex-amino 5'-NH₂-TTTTTGAGCAGAAGGGTAGTAGAGAG-3'

### First oligonucleotides (Parallel clamps):

**SEQ ID NO 4:** AA1ParallelClamp-amino: 
5'-NH2-TTTTTGAGCAGAAGGGTAGTAGAGAGTTTT-3'-3'-
CTCTCTGCTGCCCTTCTCCTC-5'
**SEQ ID NO 61:** AA1ParallelClamp: 
5'-NH2-GAGCAGAAGGGTAGTAGAGAGTTTT-3'-3'-
CTCTCTGCTGCCCTTCTCCTC -5'
**SEQ ID NO 62:** AA1ParallelClamp: 5'-NH₂-GAGCAGAAGGGTAGTAGAGAG**N**CTCTCTGCTGCCCTTCTCCTC -5" Wherein "N" is a linker molecule, preferably a single-strand nucleotide, preferably a thymidine-rich, most preferably between 3 to 5 thymidines.

### Second oligonucleotides:

**SEQ ID NO 5:** CC1biotineRP: 5'-GGCCAATAGCAAAATGACTC-BIOTINE-3'
**SEQ ID NO 5:** CC1-TamraRP: 5'-GGCCAATAGCAAAATGACTC-Tamra-3'
**SEQ ID NO 5**: CC1-Cy3RP: 5'-GGCCAATAGCAAAATGACTC-Cy3-3'
**SEQ ID NO 5:** CC1-ThioIRP: 5'-GGCCAATAGCAAAATGACTC-Thiol-3'

### DNA synthetic targets

**SEQ ID NO 6:** CC1target: 5'-GAGTCATTTTGCTATTGGCCTAGCTCTCTACTACCCTTCTGCTC-3'
**SEQ ID NO 7**: CC1-FAMtarget 5'-FAM-GCTATTGGCCTAGCT**CTCTACTACCCTTCTGCTC**GCATAGTGTATAC-3'
**SEQ ID NO 8**: CC1RNAtarget-FAM FAM-5'-CUAG**CUCUCUACUACCCUUCUGCUC**GCAUA-3'

### ➢ CC2 SYSTEM

### First oligonucleotides (PPRHs):

**SEQ ID NO 9**: CC2PPRH-amino: 
5'-NH2-
TTTTTGTGATGAGGAACGAGAAGAGGTTTTGGAGAAGAGCAAGGAGTAGT
G-3'
**SEQ ID NO 10:** CC2PPRH (gel assays): 5'-GTGATGAGGAACGAGAAGAGGTTTTGGAGAAGAGCAAGGAGTAGTG-3'
**SEQ ID NO 63**: CC2PPRH: 
5'-NH2-   GTGATGAGGAACGAGAAGAGGNGGAGAAGAGCAAGGAGTAGTG-
3'
 Wherein "N" is a linker molecule, preferably a single-strand nucleotide, preferably a thymidine-rich, most preferably between 3 to 5 thymidines.
**SEQ ID NO 11:** CC2duplex-amino: 5'-NH₂-TTTTTGTGATGAGGAACGAGAAGAGG-3'

### Second oligonucleotides:

**SEQ ID NO 12**: CC2-biotineRP: 5'- TGCGTAGAAGCCTTTTGGC-biotine-3'
**SEQ ID NO 12**: CC2-Cy3RP: 5'- TGCGTAGAAGCCTTTTGGC-Cy3-3'
**SEQ ID NO 12:** CC2-ThioIRP: 5'- TGCGTAGAAGCCTTTTGGC-thiol-3'
**SEQ ID NO 13:** CC2-biotineRPNew: 5'-CCTTCTGCGTAGAAGCCTTT -biotine-3'

### DNA synthetic targets

**SEQ ID NO 14:** CC2DNATarget: 
5’-
GCCAAAAGGCTTCTACGCAGAAGGGAGCAGAGGCGGCAGTCAAGCCTCT
TCTCGTTCCTCATCAC-3’
**SEQ ID NO 15**: CC2NewTarget:

5’-AAA
GGCTTCTACGCAGAAGGGAGCAGAGGCGGCAGTCAAGCCTCTTCTCGTTC
CTCATCAC-3’

### ➢ CC3 SYSTEM

First oligonucleotides (PPRHs):
**SEQ ID NO 16:** CC3PPRH-amino: 5'-NH₂-TTTTTGAGGGAAGGACATAAGATGATTTTAGTAGAATACAGGAAGGGAG-3'
**SEQ ID NO 17**: CC3PPRH (gel assay): 5'-GAGGGAAGGACATAAGATGATTTTAGTAGAATACAGGAAGGGAG-3'
**SEQ ID NO 64**: CC3PPRH: 5'-NH₂- GAGGGAAGGACATAAGATGANAGTAGAATACAGGAAGGGAG-3' Wherein "n" is a linker molecule, preferably a single-strand nucleotide, preferably a thymidine-rich, most preferably between 3 to 5 thymidines.
**SEQ ID NO 18**: CC3duplex-amino: 5'-NH₂-TTTTTGAGGGAAGGACATAAGATGA-3'

### Second oligonucleotides:

**SEQ ID NO 19**: CC3-biotineRPnew1: 5'-CCCTTTCCACAAAAATCAAC-BIOTINE-3'
**SEQ ID NO 19:** CC3-biotineRP: 5'- CCCTTTCCACAAAAATCAAC-biotine-3'
**SEQ ID NO 19**: CC3-Cy3RP: 5'- CCCTTTCCACAAAAATCAAC-Cy3-3'
**SEQ ID NO 19**: CC3-thioIRP: 5'- CCCTTTCCACAAAAATCAAC-thiol-3'

### DNA synthetic targets

**SEQ ID NO 20**: CC3target: 5'-GTTGATTTTTGTGGAAAGGGCTATCATCTTATGTCCTTCCCTC-3'
**SEQ ID NO 21:** CC3-FAMtarget 
FAM-5'-
GTGGAAAGGGCTATCATCTTATGTCCTTCCCTCAGTCAGCACCTCAT-3'
**SEQ ID NO 22**: CC3RNAtarget-FAM FAM-5'-GGCUA**UCAUCUUAUGUCCUUCCCUC**AGUCA-3'

### Oligonucleotides to detect RSV virus:

### First oligonucleotides (PPRHs):

**SEQ ID NO 23:** HRSV1PPRHamino: For the detection of protein N in the virion: 
5'-   NH2-AAAAGAGATGGGAGAAGTGGTTTTGGTGAAGAGGGTAGAGAAAA-
3'
**SEQ ID NO 24**: HRSV2PPRHamino: for the detection of the infection by HRSV: 5'-NH₂-AGGGTAACAAAGGAAAGGTATTTTATGGAAAGGAAACAATGGGA-3'
**SEQ ID NO 65**: HRSV1PPRH: 5'-NH₂- AAAAGAGATGGGAGAAGTGGNGGTGAAGAGGGTAGAGAAAA-3' Wherein "**N**" is a linker molecule, preferably a single-strand nucleotide, preferably a thymidine-rich, most preferably between 3 to 5 thymidines.
**SEQ ID NO 66**: HRSV2PPR: 5'-NH₂- AGGGTAACAAAGGAAAGGTANATGGAAAGGAAACAATGGGA -3' Wherein "**N**" is a linker molecule, preferably a single-strand nucleotide, preferably a thymidine-rich, most preferably between 3 to 5 thymidines.

### Second oligonucleotides:

**SEQ ID NO 25**: HRSV1RPbiotin: 5'-CAGAATATAGGCATGACTCT-biotin-3'
**SEQ ID NO 26**: HRSV2RPbiotin: 5'-AGCTTTTAATATTTGCATCG-biotin-3'

### DNA synthetic targets

**SEQ ID NO 27**: HRSV1DNAtarget: 5'-AGAGTCATGCCTATATTCTGGAGCCACTTCTCCCATCTCTTTT-3'
**SEQ ID NO 28**: HRSV2target: 5'-CGATGCAAATATTAAAAGCTTAATACCTTTCCTTTGTTACCCT-3'
**SEQ ID NO 29:** HRSV1FAMTarget FAM-5'-TGGAG**CCACTTCTCCCATCTCTTTT**AGCAT-3'
**SEQ ID NO 30:** HRSV2FAMTarget FAM-5'-CTTAA**TACCTTTCCTTTGTTACCCT**ATAAC-3'

### Oligonucleotides to detect Influenza A virus

### First oligonucleotides (PPRHs):

**SEQ ID NO 31**: CCIZ2-PPRH-amino: For the detection of HpH1N1-S2; segment 2, PB1: 
5'-NH2-
TTTTTAGAGAAAGAGACGGGTGAGAGACAATTTTAACAGAGAGTGGGCAG
AGAAAGAGA-3'
**SEQ ID NO 32**: CCIZ8-PPRH-amino: 
5’-NH2-
TTTTTGGAAGAGAAGGCAATGGTGAAATTTTAAAGTGGTAACGGAAGAGAA
GG -3’
**SEQ ID NO 55:** CCIZ2-PPRH (gel assay) 
AGAGAAAGAGACGGGTGAGAGACAAttttAACAGAGAGTGGGCAGAGAAAGA
GA
**SEQ ID NO 59:** CCIZ8-PPRH (gel assay) GGAAGAGAAGGCAATGGTGAAAttttAAAGTGGTAACGGAAGAGAAGG
**SEQ ID NO 67:** CCIZ2-PPRH 
5'-NH2-
AGAGAAAGAGACGGGTGAGAGACAANAACAGAGAGTGGGCAGAGAAAGA
GA-3'
 Wherein "N" is a linker molecule, preferably a single-strand nucleotide, preferably a thymidine-rich, most preferably between 3 to 5 thymidines.
**SEQ ID NO 68:** CCIZ8-PPRH 
5’-NH2-
GGAAGAGAAGGCAATGGTGAAANAAAGTGGTAACGGAAGAGAAGG-3’
 Wherein "N" is a linker molecule, preferably a single-strand nucleotide, preferably a thymidine-rich, most preferably between 3 to 5 thymidines.

### Second oligonucleotides:

**SEQ ID NO 33**: CCIZ2-RP-biotine: 5'-GACTAAGAAAATGATAACAC-biotin-3'

### For the detection of HpH1N1-S8; segment 8, NS1 and NEP

**SEQ ID NO 34**: CCIZ8-RP-biotine: 5'- CGCCAACAATTGCTCCCTCT-biotin-3'

### DNA synthetic targets

**SEQ ID NO 35**: CCIZ2-DNAtarget: 5'-GTGTTATCATTTTCTTAGTCATATTGTCTCTCACCCGTCTCTTTCTCT-3'
**SEQ ID NO 36:** CCIZ8-DNA Target: 5'-AGAGGGAGCAATTGTTGGCGAAATTTCACCATTGCCTTCTCTTCC-3'
**SEQ ID NO 37**: H1N1-PB1target-FAM FAM-5'-TA**TTGTCTCTCACCCGTCTCTTTCTCT**GAA-3'
**SEQ ID NO 38**: H1N1-NEPtarget-FAM FAM-5'-GAAA**TTTCACCATTGCCTTCTCTTCC**AGGA-3'

### Other sequences disclosed in the Examples

**SEQ ID NO 39:** GAGCAGAAGGGTAGTAGAGAG
**SEQ ID NO 40:** CTCTCTACTACCCTTCTGCTC
**SEQ ID NO 41:** GTGATGAGGAACGAGAAGAGG
**SEQ ID NO 42:** CCTCTTCTCGTTCCTCATCAC
**SEQ ID NO 43:** GAGGGAAGGACATAAGATGA
**SEQ ID NO 44:** TCATCTTATGTCCTTCCCTC
**SEQ ID NO 45:** CTCTCTATTACCCATCTGCTC
**SEQ ID NO 46:** CTCTACTA
**SEQ ID NO 47:** CTTATGTCCTTCCC
**SEQ ID NO 48:** TGTCCTTCC
**SEQ ID NO 49:** TACTACCCT
**SEQ ID NO 50:** CATCTTATGT
**SEQ ID NO 51:** AAAAGAGATGGGAGAAGTGGCTCCAGAATATAGGCATGACTCT
**SEQ ID NO 52:** AGGGTAACAAAGGAAAGGTA
**SEQ ID NO 53:** TACCTTTCCTTTGTTACCCT
**SEQ ID NO 54:** CGATGCAAATATTAAAAGCT
**SEQ ID NO 56:** TATGACTAAGAAAATGATAACACAG
**SEQ ID NO 57:** CTGTGTTATCATTTTCTTAGTCATA
**SEQ ID NO 58:** GTGTTATCATTTTCTTAGTC
**SEQ ID NO 69:** AGAGGGAGCAATTGTTGGCG

The invention is described below by the following examples, which must be considered as merely illustrative and in no case limiting of the scope of the present invention.

### EXAMPLES

### EXAMPLE 1: Materials and Methods

Basic protocol to design the first oligonucleotide (PPRH) adapted from Aviñó et al. 2019.

*Summary of the protocol:* This protocol describes the design of PPRHs, which are formed by a first and a second mirror-repeat polypurines regions linked by a pentathymidine bridge, and which involves the search of polypurine stretches within a given target polynucleotide. Thus, the complementary sequence of each one of these regions constitutes the polypyrimidine target region, which will be the target of the selected PPRHs. Then, the selection of sequences to build the PPRHs, among all the possible matches found in the search, is made considering the length of the sequence, the number of pyrimidine interruptions, and the specificity of that sequence within the genome of interest. Once a polypurine sequence in the target polynucleotide is selected, the mirror repeat sequence is determined by searching for its reverse sequence. Then, the final PPRH sequence is assembled by connecting these two sequences using a polythymidine linker, which will then produce a loop to allow formation of the hairpin.

*Materials:* Computer, Internet connection, Triplex-Forming Oligonucleotide Target Sequence Search software (such as the one available at http://utwl0685.utweb.utexas.edu/tfo/), Triplex target site Mapping and Integration software (such as the one available at http://ttsmi.bii.a-star.edu.sg), Reverse Complement software (such as the one available at https://www.bioinformatics.org/sms/rev comp.html), BLASTn software (such as the one available at https://blast.ncbi.nlm.nih.gov/Blast.cgi?PAGE_TYPE=BlastSearch).

### Procedure:

1. Look for polypurine sequences in the target polynucleotide using for example the TFO-searching Target Sequence Search and selecting the species and the target polynucleotide.
2. It may be required that some parameters are set by default. As an example, said parameters may be set as (1) a minimum of 12 nucleotide in length; (2) a minimum of %G: 40; and (3) a maximum allowable of 3 pyrimidine interruptions. These parameters must be changed according to specific needs of the design.
3. Select the gene or the region if you are offered a variety of genes or regions with similar names or with different transcripts and perform the search using the software.
4. Once a list of polypurine sequences is obtained, the following information may be retrieved: (1) the length of the sequence including the number and position of polypyrimidine interruptions up to 3; (2) the %G; (3) the strand of the double-stranded DNA where the polypyrimidine track is found (either forward or reverse) according to the gene orientation (in case the sequence to be targeted is found within a gene); (4) the position in the genome; and the region of the gene where it is found (promoter, intron, or exon).
5. Once the polypurine sequences are found in the target polynucleotide, it is possible to analyse the specificity of each sequence by performing a BLAST to determine the similarity of the selected sequence within the target polynucleotide. The query (input) sequence is compared with a database (e.g., nr: non-redundant) for the species of interest (e.g., coronavirus). The BLAST report generates a list of sequences in decreasing order of similarity compared to the input sequence quantified by a score followed by a selection of the sequences producing significant alignments. A polypurine sequence is considered to be specific when the report of the BLAST results in the region of interest as the most similar among all hits and there is a significant distance in score with the next sequence. This distance in score in significance depends on the length of the sequence. For instance, for a sequence of 26 nt, the difference may be -15% less, corresponding to four to five mismatches or lack of identity.
6. To design a particular PPRH out of a selected TFO target, paste that sequence into the Reverse-Complement software and determine the Reverse sequence. This sequence will correspond to the mirror repeat of the original sequence.
7. Proceed to the design of the sequence of the PPRH as follows: write the polypurine sequence followed by 4 Ts, which is then linked to the reverse sequence. The T4 will act as a loop between the two mirrored sequences that form the hairpin, which are bound by intramolecular reverse Hoogsteen bonds.
8. Design several PPRHs directed against each gene, e.g., in the promoter region, and in intron and exon sequences. Also, if possible, design template- and coding-versions of PPRHs if the target sequences exist. This will allow having a broader spectrum of PPRH sequences for screening of their potential hybridization capabilities. Regularly, it is enough to test three PPRHs to obtain at least one with the required efficiency.

### Basic protocol to design the first oligonucleotide (parallel clamp)

The method is similar as shown above from steps 1 to 8, that is, until the polypurine sequence in the target polynucleotide is defined.

9. Proceed with the design of the sequence of the parallel clamp as follows: write the polypurine sequence followed by 4 Ts, which is then linked to the polypyrimidine Hoogsteen sequence that will be in the parallel orientation. The T4 will act as a loop between the two sequences that form the clamp, which are bound by intramolecular Hoogsteen bonds. In front of G's you should add a C and in front of A you should add a T. In the interruptions in front of C you should add a G and in front of A a C (Aviñó et al 2002 Aviñó, "Properties of triple helices formed by parallel-stranded hairpins containing 8-aminopurines." Nucleic Acids Res., 30, 2609-2619)

### Basic protocol to design the second oligonucleotide (RP)

The second oligonucleotide is designed to be complementary to the adjacent viral DNA sequence. As a general method the complementary sequence is about 20 nucleotides long and starts from 3 to 15 nucleotides (to avoid self-complementarity with the clamp or hairpin sequence) after the binding of the first oligonucleotide of the clamp or hairpin.

### EXAMPLE 2. Synthesis of oligonucleotides.

Synthesis of PPRH. Oligonucleotide sequences were synthesized on an automatic Applied Biosystems 3400 DNA synthesizer on 0.2 µmοl (LV200) scale using commercially available chemicals. Oligonucleotides were prepared using standard phosphoramidite solid-phase protocols. The introduction of amino groups at the 5'-position was done by using N-trifluoroacetyl-6-aminohexyl 2-cyanoethylphosphoramidites. After the assembly of the sequences, oligonucleotide-supports were treated with 32% aqueous ammonia at 55 °C for 16 h. Ammonia solutions were concentrated to dryness and the products were purified either by cartridge purification, by Sephadex G-25 or by reversed-phase HPLC as follows. Solvent A: 5% acetonitrile in 100 mM triethylammonium acetate pH 6.5 and solvent B: 70% acetonitrile in 100 mM triethylammonium acetate pH 6.5. Columns: PRP-1 (Hamilton) or Spherisorb C18, 250 × 10 mm. Flow rate: 3 ml/min. A 30 min linear gradient from 0-50% B.

Synthesis of the parallel clamp. Similarly parallel clamps were prepared on an automatic Applied Biosystems 3400 DNA synthesizer on 0.2 µmol (LV200) scale using phosphoramidite solid-phase protocols using both standard and reversed phosphoramidites that were obtained from commercial sources. First, the polypurine sequence was assembled using standard G and A phosphoramidites. Then, the tetrathymidine loop sequence was assembled linker. Finally, The C,T-sequence was assembled using reversed C and T phosphoramidites. After the assembly of the sequences, oligonucleotide-supports were treated with 32% aqueous ammonia at 55 °C for 16 h and oligonucleotides were purified as described above for the PPRH.

Synthesis of the second oligonucleotides (in this specific case, conjugated to a reporter molecule or probe). Oligonucleotides were prepared using standard phosphoramidite solid-phase protocols. The introduction of the reporter molecule (in this case: biotin, thiol and Cy3 and TAMRA groups) was performed at the 3'-position by using controlled pore glass (CPG) functionalized with biotin-TEG, propyldisulfide and the appropriate fluorescent derivatives. All these supports were obtained from commercial sources. After the assembly of the sequences, oligonucleotide-supports were treated with 32% aqueous ammonia at 55 °C for 16 h and oligonucleotides were purified as described above for the PPRH.

### EXAMPLE 3: Design of the first and second oligonucleotides to detect SARS-CoV-2 genome and a specificity analysis of the PPRHs to be used as biosensors Design of PPRH

The starting point is one of the many SARS-CoV2 sequences available in Genbank, for instance sequence LR757995.1 containing the RNA (+) sequence. We can search for Polypurine sequences using the Triplex Forming Oligonucleotides Searching tool (Austin, Texas). Note that the sequence in this specific database is as if it were a DNA sequence, but it corresponds to the RNA of the virus.

Below it is shown an example of the search:
User File Searched: sequence.fasta.txt (LR757995.1) TFO Target Sequence Search Parameters and Results:

| **Sequences** | **length** | **%GC** | **Orientation** | **position** |
|---|---|---|---|---|
| **AGATGAGGATGAAGAAGAAGGTGA** | **24** | **41.7** | **forward** | **3031** |
| **GAGCAGAAGGGTAGTAGAGAG** | **21** | **47.6** | **reverse** | **17128** |
| **GTGATGAGGAACGAGAAGAGG** | **21** | **47.6** | **reverse** | **28823** |
| **GAGGGAAGGACATAAGATGA** | **20** | **40.0** | **reverse** | **2470** |
| **Table 1**: Results of TFO target sequences search in the SARS-CoV-2 genome. (SEQ ID NOs: 70, 39, 41, 43). | | | | |

As can be seen, the 4 sequences contain 3 pyrimidine interruptions (underlined), which are one forward and 3 reverse, as indicated in Table 1.

Since these are the polypurine sequences, the polypyrimidine sequences will be in the opposite strand. Given that the first sequence is found in the forward strand of the virus, the complementary polypyrimidine sequence will not exist, since the virus has only one strand of RNA.

Therefore, we focused in the sequences found in the reverse orientation with the longest sequence to ensure specificity, which are the 3 reverse sequences (herein after referred to as Sequence #2, Sequence #3 and Sequence #4) ranging 21-20 nucleotides (sequences 2, 3 and 4 of Table 1).

Sequence #2 is found in the polyprotein 1ab region where the replicase is encoded. Sequence #4, on the other hand, corresponds to the target found in the RNA coding for the protein Spike. Sequence #3, is located in position 28823, located in gene "N" of the virus.

Subsequently, the PPRH design will be as follows:
- For sequence #2 (CC-1): 5'-GAGCAGAAGGGTAGTAGAGAG-3' (reverse orientation), the polypyrimidine target region would be: 5'-CTCTCTACTACCCTTCTGCTC-3'.

Then, the PPRH sequence will be, including four "Ts" as connector: 5'-GAGCAGAAGGGTAGTAGAGAGttttGAGAGATGATGGGAAGACGAG-3' (named PPRH-CC1). (SEQ ID NO: 2)

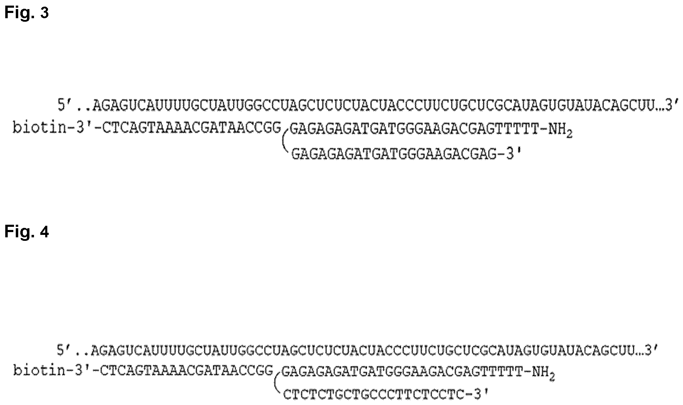

The PPRH-CC1 is binding to a Polypyrimidine target region tract within orflab. Fig. 3 shows a schematic representation of the binding of this PPRH to the genome of the virus. A second oligonucleotide conjugated to biotin at its 3' end is also depicted in Fig. 3.
- For sequence #3 (CC-2): 5'-GTGATGAGGAACGAGAAGAGG-3', whose polypyrimidine target region target is: 5'- CCTCTTCTCGTTCCTCATCAC-3'. Then, the PPRH sequence will be, including four "Ts" as connector: 5'-GTGATGAGGAACGAGAAGAGGttttGGAGAAGAGCAAGGAGTAGTG-3' (named CC2PPRH-amino). (SEQ ID NO: 10).
- For sequence #4 (CC-3): 5'- GAGGGAAGGACATAAGATGA -3', whose POLYPYRIMIDINE TARGET REGION target is: 5'- TCATCTTATGTCCTTCCCTC -3'. Then, the PPRH sequence will be, including four "Ts" as connector: 5'-GAGGGAAGGACATAAGATGAttttAGTAGAATACAGGAAGGGAG-3' (named CC3PPRH-amino, SEQ ID NO 17). The PPRH-CC1 is binding to a Polypyrimidine target region tract within the Spike coding region.

The first 2 sequences, #2 (CC-1) & #3 (CC-2), have the same %GC (47,6%), CC-1 is located in the Orf1ab of the virus, and CC-2 in N protein. Sequence #4 (CC-3) hybridizes in the Spike coding region and has a lower %GC (40%). However, it was chosen because it belongs to the Spike sequencing part of the virus. As examples, it will be continued with CC-1 and CC-3, as a representation of detection of two different parts of the virus.

### Specificity of the designed PPRH

First, we evaluated whether the PPRH designed against the polypyrimidine target regions selected above would have high hybridization specificity or whether there is the risk they hybridize in other regions of the SARS-CoV-2 genome.

Sequence #2 polypyrimidine target region: 5'-CTCTCTACTACCCTTCTGCTC-3' Blast against SARS-CoV-2: Severe acute respiratory syndrome coronavirus 2 isolate SARS-CoV-2/human/USA/FL-BPHL-1279/2020

**Table 2: BLAST analysis of the selected Sequence #2 polypyrimidine target region against SARS-CoV-2.**

| Alignment statistics for match #1 | | | | | | |
|---|---|---|---|---|---|---|
| Score | | Expect | Identities | | Gaps | Strand |
| 42.1 bits(21) | | 0.14 | 21/21(100%) | | 0/21(0%) | Plus/Plus |
| Query | 1 | CTCTCTACTACCCTTCTGCTC | | 21 | | |
| | | \| \| \| \| \| \| \| \| \| \| \| \| \| \| \| \| \| \| \| \| \| | | | | |
| Sbjct | 17085 | CTCTCTACTACCCTTCTGCTC | | 17105 | | |

Sequence #4 polypyrimidine target region: 5'- TCATCTTATGTCCTTCCCTC -3' Blast against SARS-CoV-2 : Severe acute respiratory syndrome coronavirus 2 isolate SARS-CoV-2/human/USA/FL-BPHL-1279/2020

Sequence ID: MW023472.2

Length: 29805

Number of Matches: 1

**Table 3: BLAST analysis of the selected Sequence #4 polypyrimidine target region against SARS-CoV-2.**

| Alignment statistics for match #1 | | | | | | |
|---|---|---|---|---|---|---|
| Score | | Expect | Identities | | Gaps | Strand |
| 40.1 bits(20) | | 0.54 | 20/20(100%) | | 0/20(0%) | Plus/Plus |
| Query | 1 | TCATCTTATGTCCTTCCCTC | | 20 | | |
| | | \| \| \| \| \| \| \| \| \| \| \| \| \| \| \| \| \| \| \| \| | | | | |
| Sbjct | 24665 | TCATCTTATGTCCTTCCCTC | | 24684 | | |

As can be seen, the only sequences displayed in the BLAST analysis are the two corresponding to the SARS-CoV-2 polypirimidine target regions, which would indicate that the PPRH designed to hybridize with these polypyrimidine target regions will present high affinity to these regions and would probably not hybridize in other regions of the genome.

Next, we performed further BLAST analyses of polypyrimidine target regions of sequence #2 and #4 to explore whether the PPRH designed could be targeting not only SARS-2 but also SARS-1 or even MERS viruses or whether they are completely specific to SARS-2

Sequence #2 polypyrimidine target region against SARS-CoV-1: (SARS coronavirus Tor2, complete genome).

NCBI Reference Sequence: NC_004718.3

SARS coronavirus Tor2, complete genome

Sequence ID: NC_004718.3

Length:29751

Number of Matches:49

Range 1: 17053 to 17073

**Table 4: BLAST analysis of the selected Sequence #2 polypyrimidine target region against SARS-CoV-1.**

| Alignment statistics for match #1 | | | | | | |
|---|---|---|---|---|---|---|
| Score | | | Expect | Identities | Gaps | Strand |
| 26.3 bits(13) | | | 0.004 | 19/21(90%) | 0/21(0%) | Plus/Plus |
| Query | 1 | CTCTCTACTACCCTTCTGCTC | | | 21 | |
| | | \| \| \| \| \| \| \| \| \| \| \| \| \| \| \| \| \| \| \| | | | | |
| Sbjct | 17053 | CTCTCTATTACCCATCTGCTC | | | 17073 | |

| Alignment statistics for match #2 | | | | | | |
|---|---|---|---|---|---|---|
| Score | | | Expect | Identities | Gaps | Strand |
| 16.4 bits(8) | | | 4.3 | 8/8(100%) | 0/8(0%) | Plus/Minus |
| Query | 3 | CTCTACTA | 10 | | | |
| | | \| \| \| \| \| \| \| \| | | | | |
| Sbjct | 429 | CTCTACTA | 422 | | | |

Sequence #4 polypyrimidine target region against SARS-CoV-1: (SARS coronavirus Tor2, complete genome)

NCBI Reference Sequence: NC_004718.3

SARS coronavirus Tor2, complete genome

SARS coronavirus Tor2, complete genome

Sequence ID: NC_004718.3

Length: 29751

Number of Matches: 48

Range 1: 24582 to 24595

**Table 5: BLAST analysis of the selected Sequence #4 polypyrimidine target region against SARS-CoV-1.**

| Alignment statistics for match #1 | | | | | |
|---|---|---|---|---|---|
| Score | Expect | | Identities | Gaps | Strand |
| 28.2 bits(14) | 0.001 | | 14/14(100%) | 0/14(0%) | Plus/Plus |
| Query 5 | CTTATGTCCTTCCC | | 18 | | |
| | \| \| \| \| \| \| \| \| \| \| \| \| \| \| | | | | |
| Sbjct 24582 | CTTATGTCCTTCCC | | 245 | | |

| Alignment statistics for match #2 | | | | | |
|---|---|---|---|---|---|
| Score | Expect | | Identities | Gaps | Strand |
| 18.3 bits(9) | 0.99 | | 9/9(100%) | 0/9(0%) | Plus/Minus |
| Query 9 | TGTCCTTCC | 17 | | | |
| | \| \| \| \| \| \| \| \| \| | | | | |
| Sbjct 19939 | TGTCCTTCC | 19931 | | | |

Sequence #2 polypyrimidine target region against MERS:

Blast against MERS: NC_019843.3

Middle East respiratory syndrome-related coronavirus isolate HCoV-EMC/2012, complete genome

Sequence ID: NC_019843.3

Length: 30119

Number of Matches:43

Range 1: 17099 to 17107

| Alignment statistics for match #1 | | | | | | |
|---|---|---|---|---|---|---|
| Score | | Expect | | Identities | Gaps | Strand |
| 18.3 bits(9) | | 1.1 | | 9/9(100%) | 0/9(0%) | Plus/Plus |
| Query | 6 | TACTACCCT | 14 | | | |
| | | \| \| \| \| \| \| \| \| \| | | | | |
| Sbjct | 17099 | TACTACCCT | 17107 | | | |

**Table 6:** BLAST analysis of the selected Sequence #2 polypyrimidine target region against MERS.

### Sequence #4 polypyrimidine target region against MERS:

Blast against MERS: Middle East respiratory syndrome-related coronavirus isolate HCoV-EMC/2012, complete genome

Sequence ID: NC_019843.3

Length: 30119

Number of Matches:56

Range 1: 16321 to 16330

**Table 7: BLAST analysis of the selected Sequence #2 polypyrimidine target region against MERS.**

| Alignment statistics for match #1 | | | | | |
|---|---|---|---|---|---|
| Score | | Expect | Identities | Gaps | Strand |
| 20.3 bits(10) | | 0.25 | 10/10(100%) | 0/10(0%) | Plus/Minus |
| Query | 2 | CATCTTATGT | 11 | | |
| | | \| \| \| \| \| \| \| \| \| \| | | | |
| Sbjct | 16330 | CATCTTATGT | 16321 | | |

In view of these BLAST analyses, we can conclude that the target sequence used for the PPRH design is specific for SARS-CoV2, although there is a partial similarity (90%) with SARS-1 given the similarity in their viral genome sequences. However, this sequence is very dissimilar in the case of MERS, indicating the high specificity of the detection system even when comparing sequences of other coronaviruses.

### EXAMPLE 4: Design of the first and second oligonucleotides to detect Human

### Respiratory Syncytial Virus (B Strain).

Human respiratory syncytial virus (HRSV) is the leading viral agent of serious pediatric respiratory disease worldwide. It also is responsible for substantial morbidity and mortality in the elderly and in severely immunocompromised individuals. HRSV is an enveloped non-segmented negative-strand RNA virus.

First, we performed a search of polypurine sequences in the viral cRNA of Human respiratory syncytial virus B (GenBank: KY674984.1). The following sequences corresponding to the RSV virus, using the TFO searching tool:

| **Triplex-Forming Oligonucleotide Target Sequence Search** | | | | |
|---|---|---|---|---|
| | | | | |
| **User File Searched: HRSV.txt** | | | | |

| **Sequence** | **Length** | **% G** | **Strand** | **Position** |
|---|---|---|---|---|
| **AGGAATGAGGAAAGCGAAAAAATGG** | **25** | **36.0** | **forward** | **2889** |
| **AAAAGAGATGGGAGAAGTGG** | **20** | **45.0** | **forward** | **1503** |
| **AAAGGGAAATGGGGCAAATA** | **20** | **35.0** | **forward** | **533** |
| **AGCAAGAAGAGGAAACGAAG** | **20** | **35.0** | **forward** | **6053** |
| **AGAAAAGTATGGAGATGAAG** | **20** | **35.0** | **forward** | **12590** |
| **AGGGTAACAAAGGAAAGGTA** | **20** | **35.0** | **reverse** | **14654** |
| **Table 8**: Results of TFO target sequences search in the RSV genome. (SEQ ID NOs: 71, 72, 73, 74, 75, 52) | | | | |

The first sequence is in the cRNA at the level of protein "N". Therefore, the polypyrimidine target region target will be in the "virion" sequence since it is an RNA negative virus. Thus, the PPRH to detect the "Virion" target will be: HRSV1PPRHamino (SEQ ID NO 23:):
5'-AAAAGAGATGGGAGAAGTGGttttGGTGAAGAGGGTAGAGAAAA-3'

And the probe (HRSV-V-Target), labelled with FAM for the binding analyses will be: 5'-TGGAG**CCACTTCTCCCATCTCTTTT**AGCAT-3'which corresponds to the target sequence of the PPRH with 5 nucleotides extra at each end

For the design of the second oligonucleotide we selected its hybridization region by selecting the region that is 3 nucleotides away from the polypyrimidine target region of the PPRH. Given that in the cRNA the sequence up to 23 nucleotides downstream of the PPRH target is
5'-AAAAGAGATGGGAGAAGTGGCTCCAGAATATAGGCATGACTCT-3',

The reverse complementary sequence would thus be (SEQ ID NO 27: HRSV1DNAtarget):
5'-**AGAGTCATGCCTATATTCTG**GAGCCACTTCTCCCATCTCTTTT-3'. wherein the underlined nucleotides are the three nucleotides that we count to space the first oligonucleotide (PPRH) hybridization region from the second oligonucleotide hybridization region (region in bold).

The second oligonucleotide was biotinylated at its 3' end. The sequence of said second oligonucleotide targeting N protein of RSV is (SEQ ID NO 25: HRSV1RPbiotin):
5'-CAGAATATAGGCATGACTCT-3'-Biotin

On the other hand, the polypyrimidine target region target of the reverse sequence #6 PPU 5'-AGGGTAACAAAGGAAAGGTA-3' upon Reverse complement action will be: 5'-TACCTTTCCTTTGTTACCCT-3' which will be located in the L-Pol protein and will detect the presence of the virus upon infection and Reverse transcription of its RNA. Therefore, the PPRH designed herein to detect the infection by the HRSV will be: (SEQ ID NO 24):
5'-AGGGTAACAAAGGAAAGGTAttttATGGAAAGGAAACAATGGGA-3'

And the probe (HRSV-I-Target), labelled with FAM for the binding analyses will be:
5'-CTTAA**TACCTTTCCTTTGTTACCCT**ATAAC-3' which corresponds to the target sequence of the PPRH with 5 nucleotides extra at each end

And therefore, to design the second oligonucleotide, the reverse complement to 5'-CGATGCAAATATTAAAAGCT-3' is (SEQ ID NO 26: HRSV2RPbiotin):
5'-AGCTTTTAATATTTGCATCG-3'-Biotin

### EXAMPLE 5: Design the of first and second oligonucleotides to detect Influenza virus H1N1.

### PPRH DESIGN:

Targets were located in Segment 2 and 8, by searching the TFO searching tool, as shown below:

| **Triplex-Forming Oligonucleotide Target Sequence Search** | | | | |
|---|---|---|---|---|
| | | | | |
| **User File Searched: H1N1_S2.txt** | | | | |

| **Sequence** | **Length** | **% G** | **Strand** | **Position** |
|---|---|---|---|---|
| **AGAGAAAGAGACGGGTGAGAGACAA** | **25** | **40.0** | **forward** | **581** |
| **AGGGATGCAAATAAGGGGG** | **19** | **47.4** | **forward** | **756** |
| **Table 9**: Results of TFO target sequences search in the H1N1 Influenza genome segment 2. (SEQ ID NOs: 76 and 77) | | | | |

### PPRH DESIGN:

The first sequence from the TFO target sequences search in the H1N1 Influenza genome segment 2 is used to design the corresponding PPRH:
AGAGAAAGAGACGGGTGAGAGACAAttttAACAGAGAGTGGGCAGAGAAAGAGA (HpH1N1-S2; segment 2, PB1)(Viral presence)

To design the sandwich oligonucleotide (Second oligonucleotide) the following steps are taken:
First, to determine the sequence at 3' of the Polypyrimidine target in the cRNA (SEQ ID NO: 56):
5'-TATGACTAAGAAAATGATAACACAG-3'

Then, look for its Reverse complement that would correspond to the 5'-3' in the virion (SEQ ID NO: 57):
5'-CTGTGTTATCATTTTCTTAGTCATA-3'

Leave 3 nucleotides before the polypyrimidine target region target, and make it a 20 mer (SEQ ID NO: 58):

### 5'-GTGTTATCATTTTCTTAGTC-3'

Finally, make the reverse complement of SEQ ID NO: 58 to originate the second oligonucleotide of SEQ ID NO 33: CCIZ2-RP-biotin): 5'-GACTAAGAAAATGATAACAC-3'-Biotin

| **Triplex-Forming Oligonucleotide Target Sequence Search** | | | | |
|---|---|---|---|---|
| | | | | |
| **User File Searched: H1N1_S8.txt** | | | | |

| **Sequences** | **Length** | **% G** | **Strand** | **Position** |
|---|---|---|---|---|
| **GGAAGAGAAGGCAATGGTGAAA** | **22** | **40.9** | **R** | **526** |
| **AGAAACGAGAAATGGCGGGA** | **20** | **40.0** | **F** | **679** |
| **GAAGTGGAGCAAGAGATAAG** | **20** | **40.0** | **F** | **820** |
| **Table 10**: Results of TFO target sequences search in the H1N1 Influenza genome segment 8. (SEQ ID NOs: 78, 79 and 80) | | | | |

The first sequence from the TFO target sequences search in the H1N1 Influenza genome segment 8 is used to design the corresponding PPRH:
GGAAGAGAAGGCAATGGTGAAAttttAAAGTGGTAACGGAAGAGAAGG (HpH1N1-S8; segment 8, NS1 and NEP)(infected cells) (SEQ ID: 59)

To design the sandwich oligonucleotide (Second oligonucleotide) the following steps are taken:
First, to determine the sequence at 5' of the Polypyrimidine target excluding 3 nucleotides (AAA):
5'-AGAGGGAGCAATTGTTGGCG-3'

And then make the reverse complement to originate the Sandwich-oligo (SEQ ID NO 34: CCIZ8-RP-biotine):
5'- CGCCAACAATTGCTCCCTCT-3'-Biotin

Therefore, the two PPRH designed to detect influenza are:
(HpH1N1-S2; segment 2, PB1) (Viral presence) (SEQ ID NO 55: CCIZ2-PPRH-amino): 
5´-
AGAGAAAGAGACGGGTGAGAGACAAttttAACAGAGAGTGGGCAGAGAAAGAGA
-3´
(HpH1N1-S8; segment 8, NS1 and NEP) (infected cells) (SEQ ID NO 59: CCIZ8-PPRH-amino):
   5'-GGAAGAGAAGGCAATGGTGAAAttttAAAGTGGTAACGGAAGAGAAGG-3'

### EXAMPLE 6: Binding Assays of The PPRH to their targets

Binding experiments were carried out by incubating the 6-FAM-labeled ss DNA or RNA probes corresponding to the viruses targets with the corresponding PPRHs in a buffer containing 10 mM MgCl₂, 100 mM NaCl, and 50 mM HEPES (pH 7.2), supplemented with 5% glycerol. Binding reactions (20 µL) were incubated 30 min at 37°C. Unspecific poly(dl:dC) DNA (for ss DNA probes) or tRNA (for RNA probes) was included in each reaction at a 1:1 unspecific/specific ratio. Scrambled PPRH was used as negative control. Electrophoresis was performed on nondenaturing 8% polyacrylamide gels containing 10 mM MgCl₂, 5% glycerol, and 50 mM HEPES (pH 7.2). Gels were run at a fixed voltage of 190 V (4°C) using a running buffer containing 10 mM MgCl₂ and 50 mM HEPES (pH 7.2). Finally, gels were visualized using the Gel Doc^{™} EZ with the Image Lab Software, Version 6.0 (Bio-Rad). All reagents were obtained from Sigma-Aldrich.

### Binding of PPRHs to the Sars-CoV-2 targets: ssDNA and RNA:

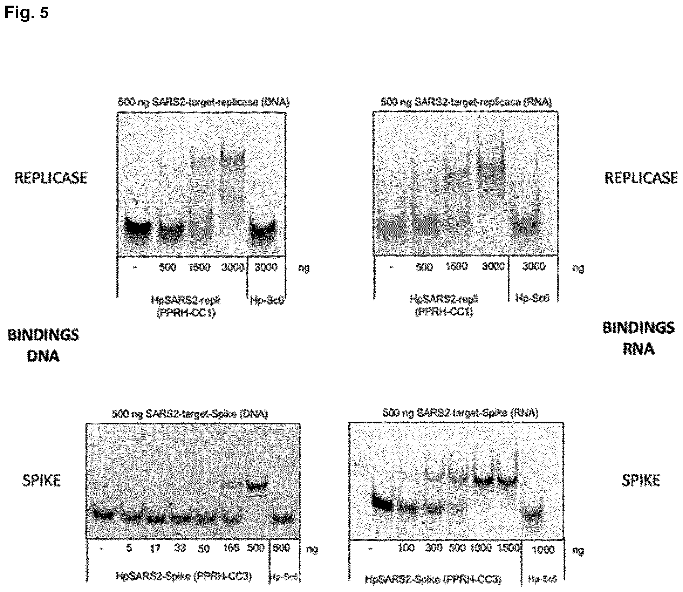

As can be seen in Fig 5, increasing amounts, as indicated, of the PPRHs designed against the viral sequences for replicase or spike were able to bind to 500 ng of their corresponding targets in the SARS2 genome, either as ssDNA or ssRNA species. In both cases a shifted band corresponding to the binding of the PPRH to its target, with a lower mobility than the probe alone, can be observed.

As a negative control a PPRH with a scrambled sequence (HpSC6) was used that did not originate any shifted band in the presence of the specific probes.

### Binding of PPRHs to the H1N1 Influenza A targets: ssDNA

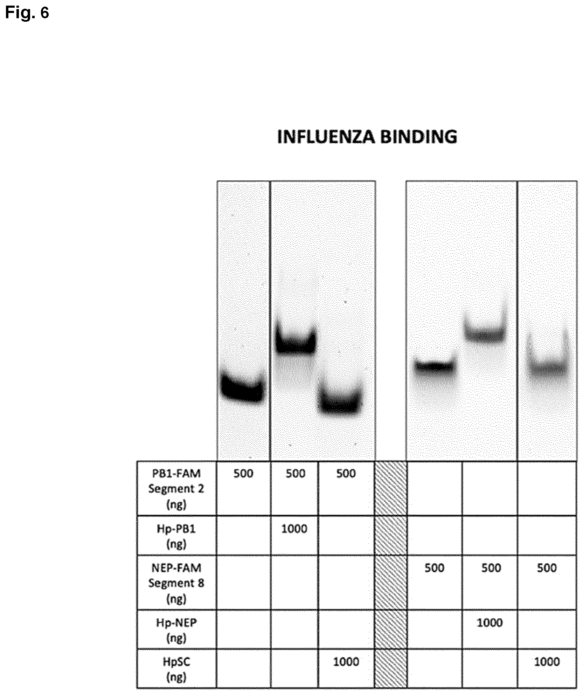

As can be seen in Fig 6, the PPRHs designed against segments 2 and 8 were able to bind to 500 ng of their corresponding targets in the Influenza A virus. In both cases a shifted band corresponding to the binding of the PPRH, either Hp-PB1 or Hp-NEP, to its target as ssDNA, with a lower mobility than the probe alone, can be observed.

As a negative control a PPRH with a scrambled sequence (HpSC) was used that did not originate any shifted band in the presence of the specific probes.

### Binding of PPRHs to the HRSV targets: ssDNA

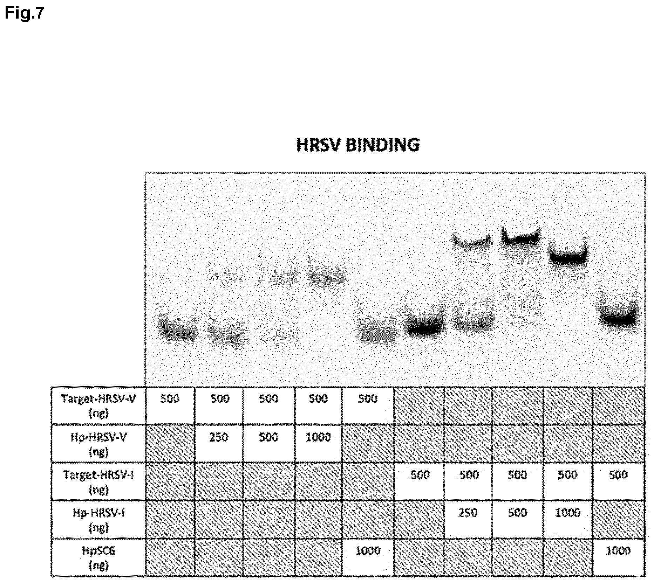

As can be seen in Fig 7, increasing amounts, as indicated, of the PPRHs designed against the HRSV sequences for the virion (HRSV-V) or its cRNA (HRSV-I) were able to bind to 500 ng of their corresponding targets. In both cases a shifted band corresponding to the binding of the PPRH to its target, with a lower mobility than the ssDNA probe alone, can be observed.

As a negative control a PPRH with a scrambled sequence (HpSC6) was used that did not originate any shifted band in the presence of the specific probes.

### EXAMPLE 7: PPRH as biosensors linked to Gold Nanoprisms

### Materials and Methods

### Gold Nanoprisms (NPrs) Synthesis

Gold NPrs with a Plasmon Band between 1000 and 1200 nm have been prepared following a methodology previously reported by Pelaz et al. [Langmuir, 2012, 28 (24), 8965-8970], prior to use, all glassware was washed with aqua regia and rinsed thoroughly with Milli-Q water from the Millipore Q-POD^{®} system from EMD Millipore (Darmstadt, Germany).

To perform the synthesis 140 mL of 0.5 mM Na₂S₂O₃ (11 mg, 70 µmol , Sigma-Aldrich, San Luis, MO, USA) aqueous solution (Milli-Q water) containing 10 µl of 0.1 M Kl (0.16 mg, 1 µmol, Panreac, Barcelona, Spain) was added slowly to 200 mL of aqueous HAuCl₄·H₂O 2 mM (136 mg, 400 µmol, Strem Chemicals, Newburyport, MA, USA) in a slow but continuous way during 30 s. After 4 min, another 140 mL of Na₂S₂O₃ 0.5 mM (11 mg, 70 µmol) containing 10 µL of Kl 0.1 M (0.16 mg, 1 µmol) were added. After another undisturbed 4 min, 60 mL of Na₂S₂O₃ 0.5 mM (4,7 mg, 30 µmol) were slowly added to the solution and the resulting mixture was left reacting for an hour at room temperature avoiding the light covering the mixture with aluminium film.

UV-VIS (Cary-50, Variant) spectra revealed a strong absorbance peak at 1000 nm corresponding to Gold Nanoprisms as well as a minor absorption band at 536 nm corresponding with the by-product Gold pseudospherical (polyhedral) nanoparticles. The concentration of NPrs was calculated using their LSPR peak absorbance at 1050 nm and applying a conversion factor (ε) 29 ml mg⁻¹ cm⁻¹. Note that ε was obtained from combined UV-VIS spectroscopy/ ICP analyses.

Gold NPrs were stabilized using heterobifunctional HS-PEG-COOH (Mercapto-w-carboxy PEG MW. 5000 Dalton, Rapp-polymere GmbH, Germany) by conjugation to the gold surface by the thiols (SH-) functional groups. For this purpose, a solution of HS-PEG₅₀₀₀-COOH (aq.) with a ratio PEG:Au NPrs of 2:1 (in mg) was added to the nanoprisms solution. HS-PEG₅₀₀₀-COOH was diluted in 1-ml Milli-Q and a determined volume of 10 mg/mL stock solution of NaBH₄ (Sigma-Aldrich, San Luis, MO, USA) was then added to reach 1:1 molar ratio of PEG:NaBH₄. After that, the pH was raised to 12 with the addition of aqueous NaOH 2M (Sigma-Aldrich, San Luis, MO, USA) under mild mixing and the solution was sonicated for 30 minutes at 60 °C to complete the reaction with HS-PEG₅₀₀₀-COOH.

The resultant mix of Gold Nanoprisms and spheres were centrifuged at 5500 rcf for 15 min at room temperature to remove unreacted reagents and unwanted by-products. While the supernatant was discarded, the precipitate was resuspended in the same volume of water and two further washing steps were performed with Milli-Q water using the same conditions.

The aqueous dispersion of PEG derivatized gold nanoprisms and gold nanospheres (2 mL, 1.5 mg/mL) were loaded (mixed with loading buffer, i.e., TBE 0.5x, 5% glycerol) in wells within an agarose gel (2.5%) immersed in an electrophoresis cuvette filled with TBE 0.5x. Electrophoresis separation was run at 120 V for 40 min. The higher electrophoretic mobility and lower hydrodynamic diameter of nanospheres compared to nanoprisms, allows the nanospheres enters in the gel and the nanoprisms stayed in the wells. The green nanoprisms solution were recovered from the wells carefully with a micropipette. The resultant dispersion of Gold nanoprisms in TBE 0.5x were centrifuged and washed with Mili-Q water (3 times in total) at 5500 rcf for 15 min at room temperature to remove the TBE buffer.

### Nanoprisms Biofunctionalization.

PEG derivatized gold nanoprisms were functionalized using amine modified oligonucleotides able to recognize specific regions of COVID-19 viral RNA (first oligonucleotides or PPRHs / second oligonucleotides or RPs). Briefly, 0.25 mg of PEG derivatized gold nanoprisms were incubated with EDC (N-(3-Dimethylaminopropyl)-N'-ethylcarbodiimide hydrochloride, Sigma-Aldrich, San Luis, MO, USA) 3 mM and Sulfo-NHS (N-Hydroxysulfosuccinimide sodium salt, Sigma-Aldrich, San Luis, MO, USA) 7mM in 0.5 mL of filtered 10 mM MES (2-Morpholinoethanesulfonic acid monohydrate, Sigma-Aldrich, San Luis, MO, USA) buffer pH 6 for 30 min at 37 °C; after that the activated gold nanoprisms were centrifuged 9 minutes at 5500 rpm. After supernatant removing the nanoprisms were incubated for 1.5 h at 37 °C with 0.68 nmol of modified oligonucleotides (PPRHs or RPs, 0.5 mL in filtered 50 mM MES buffer pH 6). Then 0.5 ml of alpha-Methoxy-omega-amino poly-ethylene glycol (MeO-PEG-NH₂ (750 Da), Rapp-polymere GmbH, Germany) in filtered 50 mM MES buffer pH 6 were added to the nanoprisms solution for 2h at 37 °C added to derivatize the remaining activated carboxylic groups. Finally, biofunctionalized nanoprims were washed out of ligand excess by centrifugation; nanoprisms were centrifuged three times for 9 min at 5500 rpm, and then pellets were resuspended in filtered 10 mM Hepes (4-(2-Hydroxyethyl)piperazine-1-ethanesulfonic acid, Sigma-Aldrich, San Luis, MO, USA) buffer pH 7,2 0.1% tween20, 0.1% BSA.

### Capture molecules (test and control lines) preparation:

**System 1:** Test line's capture molecule is prepared by dissolving Streptavidin (Streptavidin from Streptomyces avidinii, Sigma-Aldrich, San Luis, MO, USA) at 1 mg/mL in PBS 10 mM pH 7.2.

Control line's capture molecule is prepared by incubation of carboxylic acid modified oligonucleotides able to recognize the nanoprisms biofunctionalized with first oligonucleotide (PPRHs) with EDC (N-(3-Dimethylaminopropyl)-N'-ethylcarbodiimide hydrochloride, Sigma-Aldrich, San Luis, MO, USA) 1.5 mM and Sulfo-NHS (N-Hydroxysulfosuccinimide sodium salt, Sigma-Aldrich, San Luis, MO, USA) 3 mM in 0.05 mL of filtered 10 mM MES (2-Morpholinoethanesulfonic acid monohydrate, Sigma-Aldrich, San Luis, MO, USA) buffer pH 6 for 30 min at 37 °C. After that, a solution of BSA (Bovine Serum Albumin, Sigma-Aldrich, San Luis, MO, USA) 2 mg/mL (0.05 mL) in filtered 10 mM MES buffer pH 6 was added for 2 hours at 37 °C. Then, the bioconjugate was centrifuged 14 minutes at 14000 rpm using 3K Amicon Ultra (Millipore Corporation, Burlington, Massachusetts, USA) tube to concentrate it. The final concentration is determined by UV spectrophotometer at 280 nm.

System 2: Test line's capture molecule is prepared by incubation of carboxylic acid modified first oligonucleotides CC1, CC2 or CC3 (Polypurine reverse Hoogsteen hairpins (PPRHs)) able to recognize different areas in the viral RNA with EDC (N-(3-Dimethylaminopropyl)-N'-ethylcarbodiimide hydrochloride, Sigma-Aldrich, San Luis, MO, USA) 1.5 mM and Sulfo-NHS (N-Hydroxysulfosuccinimide sodium salt, Sigma-Aldrich, San Luis, MO, USA) 3 mM in 0.05 mL of filtered 10 mM MES (2-Morpholinoethanesulfonic acid monohydrate, Sigma-Aldrich, San Luis, MO, USA) buffer pH 6 for 30 min at 37 °C. After that, a solution of BSA (Bovine Serum Albumin, Sigma-Aldrich, San Luis, MO, USA) 2 mg/mL (0.05 mL) in filtered 10 mM MES buffer pH 6 was added for 2 hours at 37 °C. Then, the bioconjugate was centrifuged 14 minutes at 14000 rpm using 3K Amicon Ultra (Millipore Corporation, Burlington, Massachusetts, USA) tube to concentrate it. The final concentration is determined by UV spectrophotometer at 280 nm.

Control line's capture molecule is prepared similarly to the system 1's control line by incubation of carboxylic acid modified oligonucleotides able to recognize the nanoprisms biofunctionalized with the amine modified second oligonucleotides (RPs) with EDC (N-(3-Dimethylaminopropyl)-N'-ethylcarbodiimide hydrochloride, Sigma-Aldrich, San Luis, MO, USA) 1.5 mM and Sulfo-NHS (N-Hydroxysulfosuccinimide sodium salt, Sigma-Aldrich, San Luis, MO, USA) 3 mM in 0.05 mL of filtered 10 mM MES (2-Morpholinoethanesulfonic acid monohydrate, Sigma-Aldrich, San Luis, MO, USA) buffer pH 6 for 30 min at 37 °C. After that, a solution of BSA (Bovine Serum Albumin, Sigma-Aldrich, San Luis, MO, USA) 2 mg/mL (0.05 mL) in filtered 10 mM MES buffer pH 6 was added for 2 hours at 37 °C. Then, the bioconjugate was centrifuged 14 minutes at 14000 rpm using 3K Amicon Ultra (Millipore Corporation, Burlington, Massachusetts, USA) tube to concentrate it. The final concentration is determined by UV spectrophotometer at 280 nm.

### Preparation of lateral flow test strips.

Our LFA system is composed of 3 parts, hold together in a polyvinyl chloride (PVC) backing card sheet (Kenosha , Schweitzerlaan, The Netherlands) with a geometry of 80 mm × 300 mm: A glass fiber conjugate Pad (8964, Ahlstrom-Munksjö, Helsinki , Finland, which can be considered also as a Sample pad), a nitrocellulose membrane (FF80HP , GE Healthcare Life Sciences , UK) and a cellulose wicking pad (222, Ahlstrom-Munksjö, Helsinki, Finland) which facilize the liquid flow (Fig. 8B).

To prepare the capture and control regions, a solution of both capture and control molecules, at 1 and 3 mg/mL respectively in PBS 10 mM (Pan Biotech, GmbH, Aidenbach, Germany) were applied to the nitrocellulose membrane using a KinBio XYZ Platform Dispenser HM3030/HM3035 (Kinbio Tech.Co., Ltd., Shanghai, China). The nitrocellulose membrane was incubated after the test and control lines dispensing at 37 degrees for 1 hour in an incubator. After the test and control molecules solutions dried, the LFA strip parts are mounted onto the adhesive PVC backing card, once mounted all the system is covered by a plastic film, which ensure correct contact between parts and avoid evaporation process during the assay. The final assembly was cut into 0.4 mm wide strips and they are stored in a dry place at 4 °C.

### LFA flow strips methodology:

**System 1:** Vertical Elution: 25 µL of clinical COVID19 sample, either positive or negative, is mixed with 7,5 ng of nanoprisms functionalized with amine modified first (CC1, CC2 or CC3, PPRHs) oligonucleotides and 0,0638 nmol (26,6 µL) of a biotinylated second oligonucleotide and 23,4 µL of running buffer. This mix is preincubated during 15 minutes at room temperature. Then, 0.1 mL of mix is loaded into a well of 96 well-plate and the stripe is vertically inserted. The chromatography is performed for 15 minutes and after that the stripe is dried at 37 degrees for 15 minutes. The development of the stripes is performed using a NIR laser (1064 nm, 1200 mW, 1 minute). Positive samples will provide a dark brown spot after laser irradiation on the nitrocellulose strip (Fig. 9E). Thermochromic ink or thermosensitive paper on the back side of the nitrocellulose could be also included in order to increase the sensitivity of the sensor (Fig. 9E).

Horizontal Elution: 25µL of clinical COVID19 sample, either positive or negative, is mixed with 7,5 ng of nanoprisms functionalized with amine modified first (CC1, CC2 or CC3, PPRHs) oligonucleotides and 0,0638 nmol (26,6 µL) of a biotinylated second oligonucleotide and 23,4 µL of running buffer. This mix is preincubated during 15 minutes at room temperature, once this time ends 0.1 mL of mix is added into the strip. The chromatography is performed for 15 minutes and after that the stripe is directly developed using a NIR laser (1064 nm, 300 mW, 1 minute). Positive samples will provide a dark brown spot after laser irradiation on the nitrocellulose strip (Fig. 9A). Thermochromic ink or thermosensitive paper on the back side of the nitrocellulose could be also included in order to increase the sensitivity of the sensor (Fig. 9B).

**System 2:** Vertical Elution: 25 µL of clinical COVID19 sample, either positive or negative, is mixed with 7,5 µg of nanoprisms functionalized with amine modified second oligonucleotides (RPs) and 23,4 µL of running buffer. This mix is preincubated during 15 minutes at room temperature. Then, 0.1 mL of mix is loaded into a well of 96 well-plate and the stripe is vertically inserted. The chromatography is performed for 15 minutes and after that the stripe is dried at 37 degrees for 15 minutes. The development of the stripes is performed using a NIR laser (1064 nm, 1200 mW, 1 minute). Positive samples will provide a dark brown spot after laser irradiation on the nitrocellulose strip (Fig. 9E). Thermochromic ink or thermosensitive paper on the back side of the nitrocellulose could be also included in order to increase the sensitivity of the sensor (Fig. 9E).

Horizontal Elution: 25 µL of clinical COVID19 sample, either positive or negative, is mixed with 7,5 µg of nanoprisms functionalized with amine modified second oligonucleotides (RPs) and 23,4 µL of running buffer. This mix is preincubated during 15 minutes at room temperature, once this time ends 0.1 mL of mix is added into the strip. The chromatography is performed for 15 minutes and after that the stripe is directly developed using a NIR laser (1064 nm, 300 mW, 1 minute). Positive samples will provide a dark brown spot after laser irradiation on the nitrocellulose strip (Fig. 9C). Thermochromic ink or thermosensitive paper on the back side of the nitrocellulose could be also included in order to increase the sensitivity of the sensor (Fig. 9D).

### EXAMPLE 8: Electrochemical biosensor

Protocol that describes the different chemical reactions required to perform the hybridization assay and the detection with the electrochemical biosensor device. Materials: 200-nm diameter carboxylated magnetic nanoparticles (MNPs), EDC, MES buffer pH 5, PBS buffer pH 7.4, Tris buffer pH 7.2, citrate buffer pH 5.5, ferrocenemethanol, H₂O₂, capture molecule, second oligonucleotide conjugated with HRP reported molecule
1. Modification of magnetic nanoparticles with the first oligonucleotide (PPRH - CC1, CC2 and CC3): MNPs are washed 2 times with MES buffer and incubated with EDC linker in MES buffer. Mix thoroughly and stir for 10 min. Wash the nanoparticles two times with MES buffer and resuspend the MNPs in MES buffer. Add the capture tag and incubate for 2 h at RT under stirring conditions. Wash the MNPs 3 times with PBS and then resuspend in PBS containing 0.1 % BSA and incubate for 2 h under stirring conditions. Change the solution and resuspend the modified MNPs in PBS containing sodium azide as preservative. Store in the fridge at 4°C until use.
2. Hybridization assay: The modified MNPs with the first oligonucleotide are resuspended in Tris buffer containing 1 mM EDTA and 1M NaCl (hybridization buffer). To this solution, a certain amount of the second molecule conjugated with HRP is added together with a certain volume of the biological sample. The solution is incubated for 15 min at RT. For the calibration of the device, standard solutions with increasing concentration of the target molecule are used instead of the sample. After the incubation process, the MNPs are concentrated in PBS solution containing 0.05 % Tween 20.
3. Electrochemical detection: A small volume (less than 10 µL of the MNPs concentrated suspension is cast of each fluidic channel of the electrochemical biosensor device (Fig. 14 and Fig. 15). Solution takes between 3 and 6 min to flow and reach the area where a magnet is located to trap the MNPs. Then, a washing step is carried out by adding small volume (less than 10 µL) of citrate buffer to each fluidic channel and leave it to flow for several minutes. Then, a small volume (less than 10 µL) of citrate buffer containing H₂O₂ and ferrocenemethanol is added to each fluidic channel and leave it to flow for a short period of time (between 3 and 5 min). Then a chronoamperometric measurement is recorded with the electrochemical transducer array and the produced current signal in each channel of the electrochemical biosensor device is directly proportional to the concentration of the target molecule in the sample. Quantitative results have been achieved with the CC1 (Fig. 15), CC2 and CC3 and the minimum concentration of the respective RNA sequences has been 0.1 nM (1 fmol) in standard buffered solutions. Measurements have also been recorded in UTMs showing a decrease in the absolute current values used as the analytical signal, but the minimum concentration measured was also 0.1 nM.

### EXAMPLE 9: Fluorescent DNA microarray chip

### Preparation and protocol performance of the fluorescent DNA microarray chip

This protocol describes the chemical derivatization of the microarray slides, the spotting of the oligonucleotides, the hybridization reaction and the fluorescence signal detection

**Slide derivatization:** Plain glass slides were first cleaned by immersing them in Piranha solution (H₂SO₄:H₂O₂ 70:30 v/v) during 30 min. Then, they were rinsed with Ultrapure water, activated with 10% NaOH for 2 h and then rinsed again with ultrapure water and with ethanol 60% solution. After this cleaning step, they were dried with N₂. Next, the chemical functionalization of the slides was achieved using a silane reagent. In this sense, two methods have been developed depending on the silane reagent used, both performing equally well. The first one takes place in two steps: first, the Aminopropyltrimethoxysilane (APTMS) reacts with hydroxyl groups of the glass surface and a second step where the amino groups of the silane react with isocyanate groups of the 1,4-phenylene diisothiocyanate. The second functionalization protocol developed is based on the use of 3-(Triethoxysilyl)propyl isocyanate, reagent that allows the derivatization of the hydroxyl groups of the glass slide given that contains a terminal isocyanate group, by which the amino group of the oligonucleotides is bound. After these treatments, slides were serially washed with ethanol 60% solution, methanol and acetone dried with N₂ and stored in the dessicator until use.

**DNA microarray chip manufacture:** First of all, the first oligonucleotide solutions were prepared in filtered (0.45 µM filter) printing buffer (150 nM sodium phosphate, 0.01% SDS) and afterwards they were spotted in defined positions of the derivatized glass slides. This slide printing step was performed using a sciFLEXARRAYER S3 (Scienion), under controlled temperature (20 °C) and humidity (65%). To perform the printing of each spot 5 drops were dispensed (350 pL/drop). At the end of the whole printing process, slides were maintained for 30 min inside the microarrayer. The biofunctionalized slides could be stored at 4 °C until use. Up to 24 microarray chips could be printed in a single slide. Microarray chips for multiplex analysis consisted on a matrix of 6 × 6 spots (3 different sets of oligonucleotides (first and second oligonucleotides) including the PPRH and duplex clamp format and 3 replicates of each). This scheme could be easily modified to allow the analysis of a higher number of samples.

**Hybridization assay:** The slides were placed on an Arrayit holder provided with a silicon gasket defining 8 × 3 wells on each slide. Before starting the assay, the slides were washed (200 µl/well) three times with PBST (0.01 M phosphate buffer in a 0.8% saline solution at pH 7.5 plus 0.005% Tween 20). Target standard solutions were prepared in Hybridization buffer (10 mM Tris, 1 mM EDTA, 1 M NaCl) or in the corresponding universal transport media used to collect the samples to be analysed (7 concentration points plus zero: 0.032 nM-500 nM). Then, samples or standard solutions were added (50 µl/well) and incubated at RT for 5 min. The next hybridization step includes the addition of the second labelled (TAMRA or Cy3) oligonucleotide (50 µl/well), After an incubation of 10 min, shaking at 600 rpm at RT, the slides were again washed (200 µl/well) three times with PBST and one with ultrapure water and finally dried with N₂.

**Fluorescence signal detection:** microarray measurements were recorded on a InnoScan 710 (Innopsys) with an optical filter of 532 nm. The spots were measured by subtracting the mean of the corresponding fluorophore background intensity to the mean of the fluorophore foreground intensity using Mapix (Innopsys) software. The standard curves were analyzed with a four-parameter logistic equation using the software GraphPad Prism. Thus, the equation follows the formula: [(A-B)/1-(x/C)^{D}] + B where A is the maximal fluorescence, B the minimum, C the concentration producing 50% of the difference between A and B (IC₅₀ value), and D the slope at the inflection point of the sigmoid curve.

Fig. 11, shows a general scheme of this microarray assay already explained above, but adapted to detect SARS-CoV-2 viral RNA

### EXAMPLE 10: Single oligonucleotide pair microarray standard curves

Prior to the implementation of the multiplexed microarray platform, each oligonucleotide pair (also referred herein as CC oligonucleotide pair), i.e., the first and second labelled oligonucleotide, was tested to study if the hybridization process was occurring properly. With this aim, standard curves were constructed in assay buffer (hybridization buffer). To obtain sigmoidal curves with the best characteristic parameters (maximum and minimum absorbance, slope and limit of detection) first of all optimal concentrations of both oligonucleotides were selected to generate a fluorescence signal around 10.000-20.000 RFUs (Relative Fluorescence Units) at 70-80% of the saturation curve. For this purpose, serial dilutions of each oligonucleotide were prepared. Thus, the first oligonucleotides (PPRH and duplex clamps) were spotted at six different concentrations ranged from 1 µM to 0 using a dilution factor of 5 in printing buffer. Subsequently, eight serial dilutions of the corresponding target sequence were added (50 µl/well) ranging from 1 µM to 0 (dilution factor of 2) in Hybridization buffer. After 5 min incubation at RT to allow first hybridization step, the second oligonucleotide labeled with TAMRA (in the case of CC1 pair) or Cy3 (for CC2 and CC3 pairs) was added (50 µl/well), testing three different concentrations 500, 250 and 0 nM also prepared in Hybridization buffer. After 10 min incubation at RT and shaking at 600 rpm, the slides were washed (3 × PBST + 1 × ultrapure water), dried with N₂ and read with the microarray scanner as described in the previous example.

Fig. 12 shows the experimental design and results obtained when CC1 pair was tested by using two different formats of the first oligonucleotide: PPRH and duplex. It includes also an example of sigmoidal curve-built testing different target dilutions at specific first and second oligonucleotide concentrations. Moreover, the characteristic parameters of this specific assay are also shown. In this case the limit of detection (LoD) obtained was 0.035 pmols.

### EXAMPLE 11: Matrix effect studies on DNA microarray performance when testing different Universal transport media used for viral sample collection

Focusing on SARS-CoV-2 detection, the samples that will need to be analysed will be mainly nasopharyngeal swabs. These sort of samples are usually collected with different specimen collection swab kits containing a specimen transport tube with medium that depending on the brand can slightly vary its composition or include the inactivation agent (typically guanidine isothyocyanate) or not.

In this context, the matrix effect on the microarray performance was studied testing two different universal transport media (UTM) named buffer 1 (Deltalab + Buffer VL GeneAll) and 2 (Abbott). Buffer 1 does not contain the inactivation agent which is added (VL GeneAll) just before the RNA detection and buffer 2 that includes in its recipe guanidine thiocyanate in Tris buffer).

These studies were performed following exactly the same protocol than the one described in Example 10, but target dilutions were prepared in buffer 1 or 2 instead of hybridization buffer (see table 11).

The following table shows the results obtained:

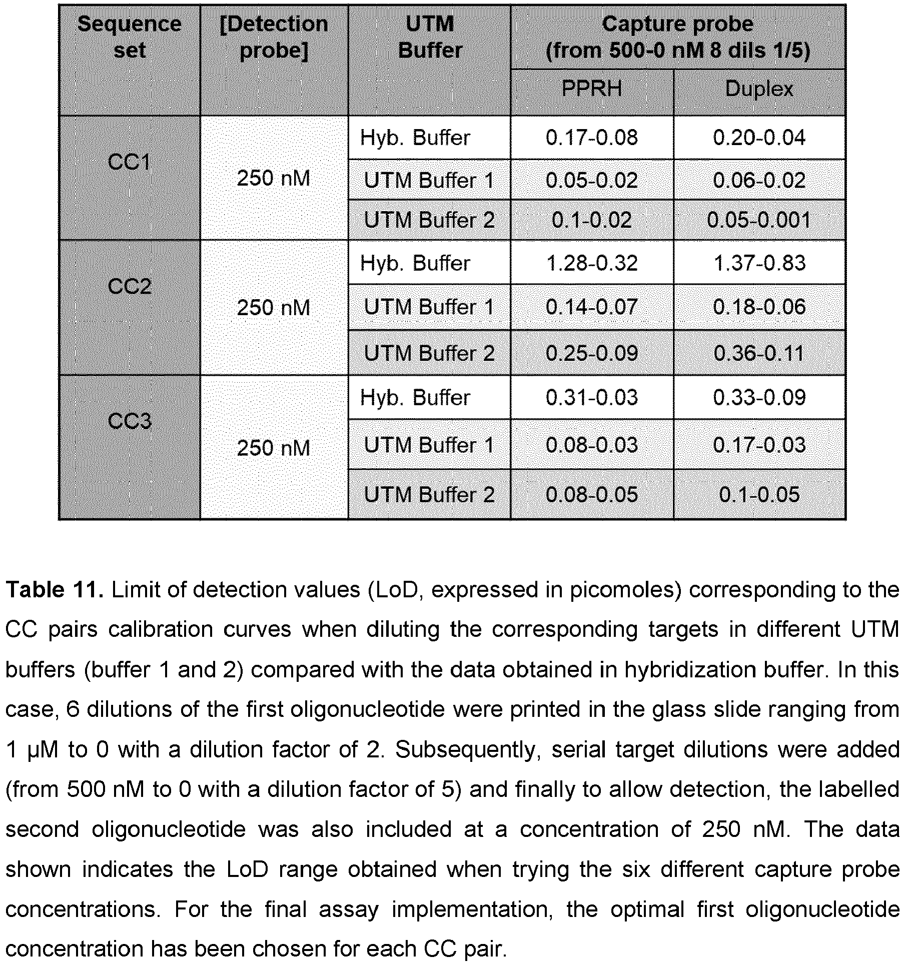

The results showed lower LoD values when the calibration curves were prepared in UTM 1 or 2 (reaching values around 1 femtomole) when compared with the values obtained with hybridization buffer

### EXAMPLE 12: Cross-reactivity determination assays and multiplexing capability analysis

DNA microarray measurements are susceptible to error caused by non-specific hybridization between a probe and a target or between probes (cross-hybridization). For this reason, a validation of the specificity of each oligonucleotide pair for its corresponding target was performed. Moreover, to identify possible nonspecific hybridization between oligonucleotides from different sets or among an oligonucleotide and a non-specific target, cross-reactivity results were of great importance.

As mentioned in previous sections, oligonucleotide design involved *in silico* BLAST and alignment analysis to select the correct sequences. Either way, to ensure reliable DNA microarray results, experimental assays were carried out analysing the fluorescence signal obtained when each target was assayed (at two different concentrations 250 and 2.5 nM) with all CC oligonucleotide pairs. Moreover, the possible cross-reactivity caused by the second oligonucleotide was also studied, by carrying out assays where the labelled probes were added at wells in which the first hybridization event had already occurred between each of the three different first oligonucleotides and the corresponding target.

These experiments showed that none of the targets neither second labelled oligonucleotides caused cross-hybridization artefacts, demonstrating that all hybridization events that take place in the microarray assay are specific.

Since one of the main advantages of microarray technology consists in presenting the possibility of detecting and quantifying several targets simultaneously, we further tested the multiplexing capability of the microarray assay developed.

With this aim, a mix of the three target sequences was tested as a reaction template (all of them added at the same concentration, performing the assay at three different target concentrations: 250, 2.5 and 0 nM). In addition, multiplexing capability was also evaluated by adding in the same well of the microarray all targets together with equal concentrations of all labelled probes (in this case two different concentrations of the second oligonucleotide were analysed: 500 and 250 nM).

## Claims

1. An *in vitro* method for detecting at least one target polynucleotide in a test sample comprising the steps of:
i) contacting the test sample with a first oligonucleotide, wherein the first oligonucleotide comprises a first polypurine region and a second region, wherein said first and second regions are connected by a linker molecule, and wherein:
a) the second region is a polypurine region and said first and second regions are capable of forming a hairpin maintained by reverse-Hoogsteen bonds with the two strands running in antiparallel orientation, or
b) the second region is a polypyrimidine region and said first and second regions are capable of forming a hairpin maintained by Hoogsteen bonds with the two strands running in parallel orientation;
wherein the first region is of at least 12 nucleotides in length and wherein at least 6 nucleotides of the at least 12 nucleotides of the first region are engaged forming said hairpin with the second region; and
wherein the first region is substantially complementary to a polypyrimidine target region comprised in the at least one target polynucleotide, wherein the hybridization between the first polypurine region and the polypyrimidine target region forms a structure comprising a triplex; and
ii) contacting the test sample with a second oligonucleotide, wherein the second oligonucleotide is substantially complementary to the at least one target polynucleotide; and
iii) detecting the hybridization between the first and the second oligonucleotides to the at least one target polynucleotide.

2. The *in vitro* method according to claim 1, wherein the second region is a polypurine region and the first and second polypurine regions are capable of forming a hairpin maintained by reverse-Hoogsteen bonds with the two strands running in antiparallel orientation and wherein the linker molecule that connects the first and the second polypurine regions is a single-stranded nucleotide region.

3. The *in vitro* method according to any of claims 1 or 2, wherein the first and the second polypurine regions comprised in the first oligonucleotide are identical in length.

4. The *in vitro* method according to any of claims 1 to 3, wherein the single-stranded nucleotide region comprised in the first oligonucleotide connects the 3'-end of the first polypurine region and the 5'-end of the second polypurine region or wherein the single-stranded nucleotide region connects the 5'-end of the first polypurine region and the 3'-end of the second polypurine region.

5. The *in vitro* method according to any of claims 1 to 4, wherein the single-stranded nucleotide region comprised in the first oligonucleotide is a thymidine-rich region, and wherein said thymidine-rich region consists of between 3 to 5 thymidines.

6. The *in vitro* method according to any of claims 1 to 5, wherein the first polypurine region comprised in the first oligonucleotide is 100% complementary to the polypyrimidine target region comprised in the at least one target polynucleotide.

7. The *in vitro* method according to any of claims 1 to 6, wherein the total length of the first oligonucleotide is between 21 to 100 nucleotides.

8. The *in vitro* method according to any of claims 1 to 7, wherein the total length of the second oligonucleotide is of at least 12 nucleotides.

9. The *in vitro* method according to any of claims 1 to 8, wherein the detection step iii) is performed by means of a reporter molecule, wherein the reporter molecule is conjugated to the first or to the second oligonucleotide.

10. The *in vitro* method according to any of claims 1 to 9, wherein the at least one target polynucleotide detected is a viral polynucleotide.

11. The *in vitro* method according to claim 10, wherein the viral polynucleotide is single-stranded, preferably RNA single-stranded.

12. The *in vitro* method according to claim 11, wherein the viral polynucleotide is selected from the group consisting of coronavirus, Influenza virus, or Respiratory syncytial virus genomes, or a combination thereof.

13. The *in vitro* method according to any of claims 1 to 12, wherein the test sample is an isolated biological sample, preferably selected from the group consisting of saliva, mucus, nasopharyngeal swabs or washings from bodily cavities.

14. The *in vitro* method according to any of claims 1 to 13, wherein the *in vitro* method further comprises a step iv) of quantifying the amount of target polynucleotide present in the test sample.

15. A system for detecting a target polynucleotide in a test sample comprising:
i) a first oligonucleotide, wherein the first oligonucleotide comprises a first polypurine region and a second region, wherein said first and second regions are connected by a single-stranded nucleotide region, and wherein:
a) the second region is a polypurine region and said first and second regions are capable of forming a hairpin maintained by reverse-Hoogsteen bonds with the two strands running in antiparallel orientation, or
b) the second region is a polypyrimidine region and said first and second regions are capable of forming a hairpin maintained by Hoogsteen bonds with the two strands running in parallel orientation;
wherein the first region is of at least 12 nucleotides in length and wherein at least 6 nucleotides of the at least 12 nucleotides of the first region are engaged forming said hairpin with the second region; and
wherein the first region is substantially complementary to a polypyrimidine target region comprised in the at least one target polynucleotide, wherein the hybridization between the first polypurine region and the polypyrimidine target region forms a structure comprising a triplex; and
ii) a second oligonucleotide, wherein the second oligonucleotide is substantially complementary to the at least one target polynucleotide; and
iii) optionally, a reporter molecule.
